Europäisches Patentamt

European Patent Office

Office européen des brevets

(19)

(11) Publication number: **0 268 772 B1**

# EUROPEAN PATENT SPECIFICATION

(12)

(45) Date of publication of patent specification: **26.04.95**

(51) Int. Cl.6: **C12N 9/10**, C12N 15/54, C12N 15/62, C12N 1/18, A61K 38/45

(21) Application number: **87113679.2**

(22) Date of filing: **18.09.87**

(83) Declaration under Rule 28(4) EPC (expert solution)

(54) **Expression of biologically active factor XIII.**

(30) Priority: **19.09.86 US 909512**

(43) Date of publication of application:
**01.06.88 Bulletin 88/22**

(45) Publication of the grant of the patent:
**26.04.95 Bulletin 95/17**

(84) Designated Contracting States:
**AT BE CH DE FR GB IT LI LU NL SE**

(56) References cited:
**EP-A- 0 011 739**
**EP-A- 0 164 556**
**EP-A- 0 189 998**
**EP-A- 0 236 978**
**DE-A- 3 621 371**

**BIOCHEMISTRY, vol. 13, no. 4, 1974, pages 750-756; T. TAKAGI et al.: "Amino acid sequence studies on Factor XIII and the peptide released during its activation by thrombin"**

(73) Proprietor: **ZymoGenetics, Inc.**
**1201 Eastlake Avenue East**
**Seattle**
**Washington 98102 (US)**

Proprietor: **THE BOARD OF REGENTS OF THE UNIVERSITY OF WASHINGTON**
**3755 University Way Northeast**
**Seattle,**
**Washington 98195 (US)**

(72) Inventor: **Davie, Earl W.**
**9010 N.E. 22nd Place**
**Bellevue, Washington 98004 (US)**
Inventor: **Seale, Ronald L.**
**611 Sixteenth Avenue East**
**Seattle, Washington 98112 (US)**
Inventor: **Ichinose, Akitada**
**4877 Terrace Drive N.E.**
**Seattle, Washington 98145 (US)**
Inventor: **Holly, Julie Ann**
**9721-2nd Avenue N.W.**
**Seattle, Washington 98117 (US)**

BIOCHEMISTRY, vol. 25, no. 16, 12th August 1986, pages 4633-4638, American Chemical Society; A. ICHINOSE et al.: "Amino acid sequence of the b subunit of human factor XIII, a protein composed of ten repetitive segments"

CHEMICAL ABSTRACTS, vol. 103, 1985, page 115, no. 33009h, Columbus, Ohio, US;Y. LI et al.: "Expression of the HO gene under control of the GALIO promoter inyeast" & YICHUAN XUEBAO 1985, 12(1), 1-6

THE PROCEEDINGS OF BIOTECH '84 EUROPE, vol. 1, pages 505-517; A. BOLLEN:"Cloning and expression in micro-organisms of the genetic information coding for human blood proteins: a powerful way to aid replacement therapy"

BIOTECHNOLOGY GENETIC ENGINEERING REVIEW, vol. 3, 1985, pages 377-416, Intercept Ltd; S.M. KINGSMAN et al.: "Heterologous gene expression in Saccharomyces cerevisiae"

BIOCHEMISTRY, vol. 25, no. 22, 4th November 1986, pp. 6900-6906, American Chemical Society; A. ICHINOSE et al.: "Amino acid sequence of the a subunit ofhuman factor XIII"

PROCEEDINGS OF THE NAT. ACADEMY OF SCIENCES OF USA, vol. 83, no. 21, November 1986, pages 8019-8023, Washington, DC, US; N. TAKAHASHI et al.: "Primary structure of blood coagulation factor XIIIa (fibrinoligase, transglutaminase) from human placenta"

Inventor: **Parker, Gary E.**
**170 South Meadowlark Road**
**Coupeville, Washington 98239 (US)**

(74) Representative: **Brown, John David et al**
**FORRESTER & BOEHMERT**
**Franz-Joseph-Strasse 38**
**D-80801 München (DE)**

**Description**

Technical Field

The present invention relates to the production of Factor XIII and its analogs in general, and more specifically, to the expression of biologically active Factor XIII and analogs thereof in transformed or transfected host cells.

Background Art

Blood coagulation is a process consisting of a complex interaction of various blood components or factors which eventually gives rise to a fibrin clot. Generally, the blood components that participate in what has been referred to as the coagulation "cascade" are proenzymes or zymogens, enzymatically inactive proteins that are converted to proteolytic enzymes by the action of an activator, itself an activated clotting factor. Coagulation factors that have undergone such a conversion are generally referred to as "activated factors," and are designated by the addition of a lower case "a" (e.g., XIIIa).

One component of the blood coagulation system is Factor XIII (also known as fibrin stabilizing factor, fibrinoligase, or plasma transglutaminase), a plasma glycoprotein that circulates in blood as a proenzyme. During the final stages of blood coagulation, thrombin converts the proenzyme form of Factor XIII to an intermediate form, which then dissociates in the presence of calcium ions to produce the active form of the enzyme, designated Factor XIIIa. Thrombin catalyzes the polymerization of fibrin through $\gamma$-dimerization followed by $\alpha$-polymerization, resulting in a non-covalent fibrin clot, which is converted to a crosslinked clot of considerable mechanical strength by Factor XIIIa (R. Chen & R.F. Doolittle, Proc. Natl. Acad. Sci. USA 66:472-479, 1970; . J.J. Pisano et al., Ann. N.Y. Acad. Sci. 202:98-113, 1972). Factor XIIIa is a transglutaminase that catalyzes the crosslinking of fibrin polymers through the formation of intermolecular $\epsilon(\gamma$-glutamyl)lysine bonds. This crosslinking reaction requires the presence of calcium ions (L. Lorand et al., Prog. Hemost. Thromb. 5:245-290, 1980; J.E. Folk and J.S. Finlayson, Adv. Prot. Chem. 31:1-133, 1977).

In vivo, Factor XIIIa catalyzes crosslinking reactions between other protein molecules. For instance, Factor XIIIa also catalyzes the crosslinking of the $\gamma$ chain of fibrin to $\alpha_2$-plasmin inhibitor and fibronectin, as well as the crosslinking of collagen and fibronectin, which may be related to wound healing (Y. Sakata and N. Aoki, J. Clin. Invest. 65:290-297, 1980; D.F. Mosher, J. Biol. Chem. 250:6614-6621, 1975; D.F. Mosher and P.E. Chad, J. Clin. Invest. 64:781-787, 1979; Folk and Finlayson, ibid.; Lorand et al., ibid.). The covalent incorporation of $\alpha_2$-plasmin inhibitor into the fibrin network may increase the resistance of the clot to lysis (Lorand et al., ibid.).

In plasma, the best amino group donor and amino group acceptor for Factor XIIIa are $\alpha_2$-plasmin inhibitor and fibrin, respectively (T. Tamaki and N. Aoki, J. Biol. Chem. 257:14767-14772, 1982; F. Carmassi and S.I. Chung, Prog. Fibrinolysis 6:281-285, 1983). Deficiencies in either Factor XIII or $\alpha_2$-plasmin inhibitor result in "delayed bleeding," while primary hemostasis in these individuals is normal (Lorand et al., ibid.). Thus, a role for both Factor XIII and $\alpha_2$-plasmin inhibitor in the protection of fibrin clots from digestion by plasmin is suggested.

Factor XIII ($M_r = \sim$320 kD) zymogen circulates in the blood as a complex with fibrinogen (C.S. Greenberg and M.A. Shuman, J. Biol. Chem. 257:6096-6101, 1982). Factor XIII is a tetramer ($a_2 b_2$) consisting of two a subunits ($M_r = \sim$75 kD) and two b subunits ($M_r = \sim$80 kD) (S.I. Chung et al., J. Biol. Chem. 249:940-950, 1974). The a subunit contains the catalytic site of the enzyme, while the b subunit is thought to stabilize the a subunit or to regulate the activation of Factor XIII (Folk and Finlayson, ibid.; L. Lorand et al., Biochem. Biophys. Res. Comm. 56:914-922, 1974). During the activation of Factor XIII to Factor XIIIa, cleavage by thrombin results in release of an activation peptide ($M_r = 4$ kD) from the amino-terminus of each of the a subunits (Schwartz et al., J. Biol. Chem. 248: 1395-1407, 1973). The amino acid sequences of the activation peptide and the region of the a chain containing the catalytic site have been determined (T. Takagi and R.F. Doolittle, Biochem. 13:750-756, 1974; J.J. Holbrook et al., Biochem. J. 135:901-903, 1973).

Current treatment practices for patients having Factor XIII deficiencies generally involve replacement therapy with plasma or plasma derivatives, or with a crude placental Factor XIII concentrate (Lorand et al., ibid.; Frobisch et al., Dtsch. Med. Wochenschr. 97:449-502, 1972; Kuratsuji et al., Haemostasis 11:229-234, 1982). This concentrate requires the use of a relatively large amount of diverse human plasma or placental tissue as starting material. Further, it is difficult to ensure that concentrates obtained from pooled human placental tissue are free from viral and other contamination. In addition, the purification of large amounts of placental Factor XIII concentrate is difficult and expensive.

Recently, a Factor XIII concentrate has been used in patients with disorders in postoperative wound healing (Mishima et al., Chirurg 55:803-808, 1984; Baer et al., Zentrabl. Chir. 105:642-651, 1980) and scleroderma (Delbarre et al., Lancet 2:204, 1981). Furthermore, Factor XIII has been used as a component of tissue adhesives (U.S. Patents 4,414,976; 4,453,939; 4,377,572; 4,362,567 and 4,298,598) and has been suggested for use in antifibrinolytic therapy for the prevention of postoperative bleeding and in the treatment of subarachnoid hemorrhage, ulcerative colitis and general wound healing. These potential applications of human Factor XIII preparations provide additional incentives to the production of recombinant Factor XIII.

Consequently, there exists a need in the art for a method of producing relatively large quantities of pure preparations of Factor XIII. The present invention fulfills this need through the use of recombinant DNA technology, successfully eliminating the problem of viral contamination and, at the same time, providing a consistent, homogeneous, and economical source of active Factor XIII to treat Factor XIII-deficient patients and others.

## Disclosure of the Invention

Briefly stated, the present invention discloses DNA sequences that code for proteins having substantially the same biological activity as Factor XIII.

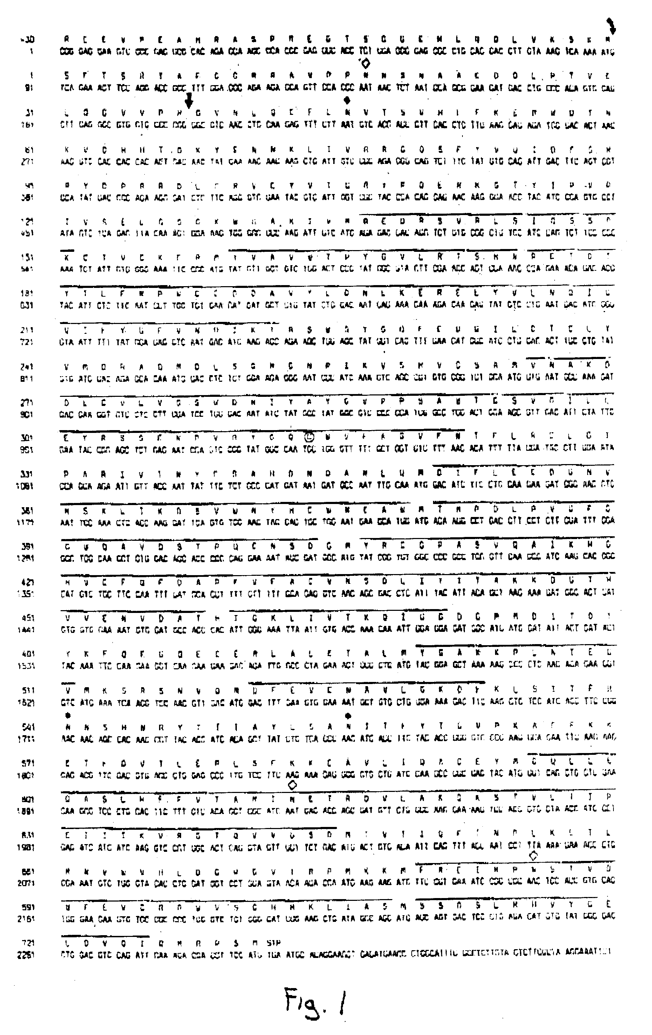

The invention provides a DNA sequence that codes for a polypeptide that is functionally homologous to the a' subunit of Factor XIII, which sequence comprises sequence of Figure 1, from bp 202 to bp 2283, as well as a DNA sequence that codes for a polypeptide that is functionally homologous to the a' subunit of Factor XIII, which sequence comprises a DNA sequence coding for the amino acid sequence of Figure 1, starting with glycine, number 38, and ending with methionine, number 731.

In addition, an expression vector comprising a transcriptional promoter followed downstream by a DNA sequence according to the preceding paragraph is also disclosed and claimed.

Host cells transfected with an expression vector according to the preceding paragraph are also disclosed. The host cell may be a prokaryotic host cell or a eucaryotic host cell, yeast host cells and mammalian host cells being particularly preferred eucaryotic host cells.

The present invention also provides a method of preparing a protein which, upon dissociation, has the biological activity of Factor XIIIa, comprising:

introducing into a host cell an expression vector comprising a transcriptional promoter followed downstream by a DNA sequence that codes for a polypeptide that is functionally homologous to the a' subunit of Factor XIII comprising the amino acid sequence of Figure 1, starting with glycine, number 38, and ending with methionine, number 731, and a transcriptional promoter followed downstream by a DNA sequence that codes for a polypeptide that is functionally homologous to the b subunit of Factor XIII,

culturing said host cell; and

isolating the protein from said host cell, as well as a method of preparing a protein having the biological activity of Factor XIIIa, comprising:

introducing into host cells an expression vector comprising a transcriptional promoter followed downstream by a DNA sequence that codes for a polypeptide that is functionally homologous to the a' subunit of Factor XIII comprising the amino acid sequence of Figure 1, starting with glycine, number 38, and ending with methionine, number 731;

culturing said host cells;

isolating polypeptide from said host cells; and

dimerizing the polypeptide to form homodimers.

The present invention also provides an expression vector comprising a transcriptional promoter followed downstream by a DNA sequence that codes for a protein having the structure and biological activity of human Factor XIII a subunit or the human Factor XIII a'subunit comprising the amino acid sequence of Figure 1, starting with glycine, number 38, and ending with methionine, number 731, wherein said DNA sequence is essentially free of 3' untranslated sequences normally associated with said DNA sequence; as well as a host cell transfected with an expression vector comprising a transcriptional promoter followed downstream by a DNA sequence that codes for a protein having the structure and biological activity of human Factor XIII a subunit or the human Factor XIII a' subunit comprising the amino acid sequence of Figure 1, starting with glycine, number 38, and ending with methionine, number 731, wherein said DNA sequence is essentially free of 3' untranslated sequences normally associated with said DNA sequence; and a method of preparing a protein having the biological activity of human Factor XIII, comprising: introducing into a host cell an expression vector comprising a transcriptional promoter followed downstream by a DNA sequence that codes for a protein having the structure and biological activity of human Factor XIII a subunit or the human Factor XIII a' subunit comprising the amino acid sequence of Figure 1, starting with glycine, number 38, and ending with methionine, number 731, wherein said DNA sequence is essentially free of 3' untranslated sequences normally associated with said DNA sequence; culturing said host cell; and isolating the protein encoded by said DNA sequence from said host cell.

In addition to disclosing proteins produced according to the methods briefly described above, the present invention is also directed towards a variety of pharmaceutical compositions comprising an effective amount of a protein produced according to each of the methods briefly described above, and a physiologically acceptable carrier or diluent.

Other aspects of the invention will become apparent upon reference to the following detailed description and attached drawings.

Brief description of the drawings

Figure 1 illustrates the nucleotide sequence of the cDNA coding for the a and a' subunits of human Factor XIII and the predicted amino acid sequences of these subunits. The amino acids numbered -30 to -1 appear to represent an in-frame sequence, while those numbered +1 (serine) to +731 (methionine) represent the amino acids present in the mature a subunit. The amino acids numbered +38 (glycine) to +731 (methionine) represent the amino acids present in the mature a' subunit.

The active site Cys at residue +315 is circled. The curved arrow shows the site of cleavage by a processing protease (such as a MET amino peptidase) generating the mature protein with an amino-terminal Ser. The straight arrow identifies the cleavage site for the conversion of the Factor XIII a subunit to the Factor XIII a' subunit by thrombin. Residues marked with solid diamonds are potential Asn-linked glycosylation sites at Asn-X-Ser or Asn-X-Thr sequences. The open diamonds identify Asn residues with little or no carbohydrate.

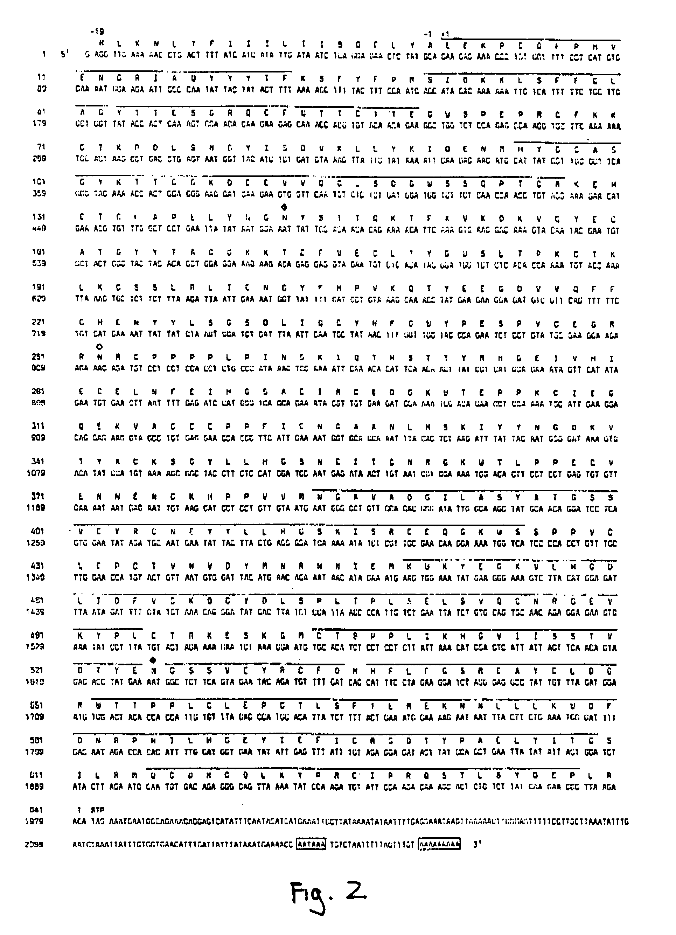

Figure 2 illustrates the nucleotide sequence of the cDNA coding for the b subunit of human Factor XIII and the predicted amino acid sequence. The amino acids numbered -19 to -1 represent a portion of a leader sequence, while those numbered +1 (glutamate) to +641 (threonine) represent the amino acids present in the mature b subunit.

The amino acid residues that are overlined were also determined by amino acid sequence analysis. (Residues 394, 536, 537, however, were not identified by this analysis.) Residues marked with solid diamonds are potential Asn-linked glycosylation sites at Asn-X-Ser sequences, while the residue marked with an open diamond is a potential Asn-linked glycosylation site at an Asn-X-Cys sequence. The nucleotide sequences that correspond to the polyadenylation signal and the poly(A) tail are shown in boxes.

Figure 3 illustrates a comparison of the amino acid sequence in repeats 4, 5 and 6 of the Factor XIII b subunit with the five repeats in the $\beta_2$-glycoprotein I, the three repeats in the Ba chain of factor B of human complement, and human haptoglobin $\alpha^1$ chain. Four or more identical residues in the same position are boxed. Gaps were inserted to obtain maximum alignment. The numbers prior to each repeat identify the residue number of the first amino acid in each repeat.

Figure 4 illustrates the alignment of the ten amino acid repeats present in the b subunit of Factor XIII. The repeats were subclassified into four groups including repeats 1, 2, and 3; 4, 5, and 6; 7 and 9; and 8 and 10. The numbers prior to each repeat identify the residue number of the first amino acid in each repeat. Two or more identical residues at the same position in each group of repeats were boxed. Gaps were inserted to obtain maximal alignment between the ten repeated segments.

Figure 5 illustrates the construction of plasmid pMVR1.

Figure 6 illustrates the construction of plasmid pAT-1.

Figure 7 illustrates the construction of plasmid pTRK4c and its derivatives pRS185.

Figure 8 illustrates the construction of pRS201 and the mutageneses that led to the construction of plasmids pRS202 and pRS203.

Figure 9 illustrates the construction of the yeast expression vectors pRS216 and pRS217.

Figure 10 illustrates the construction of plasmid pRS111, comprising the codon optimized MFα1 signal peptide sequence with the natural Glu-Ala-Glu-Ala and Hind III sequence.

Figure 11 illustrates the construction of pRS220 and pRS231.

Best Mode for Carrying Out the Invention

Prior to setting forth the invention, it may be helpful to an understanding thereof to set forth definitions of certain terms to be be used hereinafter.

Biological Activity: A function or set of functions performed by a molecule in a biological context (i.e., in an organism or an in vitro facsimile). Biological activities of proteins may be divided into catalytic and effector activities. Catalytic activities of clotting factors generally involve the activation of other factors through the specific cleavage of precursors. Effector activities include specific binding of the biologically active molecule to calcium or other small molecules, to macromolecules such as proteins, or to cells. Effector activity frequently augments, or is essential to, catalytic activity under physiological conditions. Catalytic and effector activities may, in some cases, reside within the same domain of a protein.

As noted above, the biological activity of human Factor XIII is characterized by its ability to stabilize blood clots by crosslinking fibrin polymers through the formation of intermolecular E($\gamma$-glutamyl) lysine bond. Factor XIII can also crosslink fibrin to other molecules, such as $\alpha_2$-plasmin inhibitor. Factor XIII zymogen is a tetramer composed of four subunits, two a subunits and two b subunits, that are joined by noncovalent interactions. Interchain disulfide bonds may be absent from the a subunit, although the b subunit appears to contain 20 intrachain disulfide bonds.

Factor XIII is activated through a two-step reaction. First, thrombin cleaves a short peptide (activation peptide) from the amino-terminus of each of the a subunits, resulting in the structure $a'_2 b_2$. This intermediate structure dissociates in the presence of calcium ions, yielding the inactive $b_2$ subunit and the catalytic $a'_2$ subunit (R.D. Cooke, Biochem J. 141:683-691, 1974). The b subunits are believed to protect or stabilize the a subunits, and are present in excess in plasma. The $a_2$ subunit is found within megakaryocytes, platelets, placenta, uterus, spleen, prostate, and macrophages (S.I. Chung, ibid.; T.H. Kiesselbach and R.H. Wagner, Ann. N.Y. Acad. Sci. 202:318-328, 1972; P. Henriksson et al., J. Clin. Invest. 76:528-534, 1985; L. Muszbek et al., Thromb. Res. 37:401-410, 1985). Endogenous b subunits appear to stabilize infused placental Factor XIII in the plasma.

Neither the a nor b subunit of Factor XIII has been extensively sequenced by others skilled in the art prior to the efforts described herein. The sequence of the activation peptide released from the a subunit, as well as the amino acid sequence of the region around the catalytic site of the a subunit, have been previously reported (Takagi & Doolittle, ibid.; Holbrook et al., ibid.). The only amino acid sequence information previously available for the b subunit of Factor XIII is the amino-terminal sequence Glu-Glx-Lys-Pro (Takagi and Doolittle, ibid.). Thus, Factor XIII is very poorly characterized as compared to other blood coagulation factors. As a result, very little is known about the genes encoding Factor XIII.

The present invention discloses DNA sequences that code for polypeptides that are functionally homologous to the a, a', and b subunits of human Factor XIII. The complete a and b subunits of human Factor XIII and the corresponding amino acid sequences of the a and b subunits are disclosed. The a subunit lacks disulfide bonds and contains very little, if any, carbohydrate. In addition, one post-translational modification of Factor XIII is the acetylation of the amino-terminus of the a chain. Biologically active Factor XIII may be expressed in prokaryotic as well as eukaryotic cells.

Human Factor XIII cDNA may be isolated from a human cDNA library made from an appropriate source of messenger RNA. One source of a subunit mRNA is human placental cells. A cDNA library from this source may be screened with affinity-purified antibody against the a subunit of human Factor XIII, and/or with an oligonucleotide probe that corresponds to an amino acid sequence of the activation peptide. A preferred source of b subunit mRNA is human liver mRNA. A cDNA library from this source may be screened with affinity-purified antibody against the a subunit of human Factor XIII. If incomplete cDNA clones are identified through this screening procedure, it may be desirable to rescreen the cDNA library using the 5' or 3' ends of partial clones.

In one embodiment of the present invention, it may be desirable to substitute serine for cysteine within the amino acid sequence of Factor XIII. The presence of free sulfhydryl groups may lead to oxidation of Factor XIII, and thus produce an undesirable instability of the protein. In general, the serine may be substitued for the cysteine using oligonucleotide-directed site-specific mutagenesis, as described, for example, by Zoller et al. (Manual for Advanced Techniques in Molecular Cloning Course, Cold Spring Harbor Laboratory, 1983; Zoller and Smith, DNA 3:479-488, 1984; Kunkel et al. Proc. Natl. Acad. Sci. USA 82:488-492, 1985). Polymorphic differences within the DNA sequences may exist, and therefore the sequences set forth herein provide an example of one allele. It should be noted, however, that these polymorphic differences still result in polypeptides that are functionally homologous to the a, a', and b subunits of Factor XIII. The a chain of Factor XIII may be produced cytoplasmically (vs. secreted) in yeast host cells, due to the ability of yeast cells to acetylate amino terminal serine residues of cytoplasmic proteins and due to the paucity of disulfide bonds in the a chain. However, secretion of the Factor XIII a subunit from yeast host cells will facilitate the purification of the protein.

When a full-length DNA sequence encoding a polypeptide that is functionally homologous to the a subunit, a' subunit, or the b subunit of human Factor XIII has been obtained, the DNA sequence is then inserted into a suitable expression vector. Expression vectors useful in carrying out the present invention will further comprise a promoter operably linked to the DNA sequence encoding a, a′, or b polypeptides. It is preferred that expression vectors further comprise an origin of replication, as well as nucleotide sequences which regulate and/or enhance expression levels, depending on the host cell selected. Suitable expression vectors may be derived from plasmids or viruses, or may contain elements of both. The selection of suitable vectors and their component nucleotide sequences will be evident to one of ordinary skill in the art.

The a or a′ subunit may also be co-expressed with the b subunit in a recombinant cell. To this end, two expression units are introduced into the host cell. The expression units may be on different vectors with different selectable markers or, preferably, on a single expression vector. The latter strategy offers the advantage of providing equal copy numbers of the two expression units.

Preferred prokaryotic host cells for use in carrying out the present invention are strains of the bacteria Escherichia coli, although Bacillus and other genera are also useful. Techniques for transforming these hosts and expressing foreign genes cloned in them are well known in the art (see, e.g., Maniatis et al., Molecular Cloning: A Laboratory Manual, Cold Spring Harbor Laboratory, 1982). Vectors used for expressing foreign genes in bacterial hosts will generally contain a selectable marker, such as a gene for antibiotic resistance, and a promoter which functions in the host cell. Appropriate promoters include the trp (Nichols and Yanofsky, Meth. Enzymol. 101:155-164, 1983), lac (Casadaban et al., J. Bacteriol. 143:971-980, 1980), and phage λ promoter systems (Queen, J. Mol. Appl. Genet. 2:1-10, 1983). Plasmids useful for transforming bacteria include pBR322 (Bolivar et al., Gene 2:95-113, 1977), the pUC plasmids (Messing, Meth. Enzymol. 101:20-77, 1983; Vieira and Messing, Gene 19:259-268, 1982), pCQV2 (Queen, ibid.), and derivatives thereof. Plasmids may contain both viral and bacterial elements.

Eukaryotic microorganisms, such as the yeast Saccharomyces cerevisiae, Schizosaccharomyces pombe or filamentous fungi (e.g., Aspergillus spp., Neurospora spp.) may also be used as host cells within the present invention. Techniques for transforming yeast are well known in the literature, and have been described, for instance, by Beggs (Nature 275:104-108, 1978). Aspergillus species may be transformed according to known procedures, for example, that of Yelton et al. (Proc. Natl. Acad. Sci. USA 81:1740-1747, 1984). Suitable yeast expression vectors include YRp7 (Struhl et al., Proc. Natl. Acad. Sci. USA 76:1035-1039, 1979), YIP5 (Struhl et al., ibid.), YEp13 (Broach et al., Gene 8:121-133, 1979), pJDB248 and pJDB219 (Beggs, ibid.), and derivatives thereof. Such vectors will generally include a selectable marker, such as the nutritional marker LEU2, which allows selection in a host strain carrying a leu2 mutation or may include an "essential gene" as a selectable marker (Kawasaki and Bell, EP 171,142). A preferred such essential gene marker is the POT1 gene of Schizosaccharomyces pombe, which provides for stable plasmid maintenance in a TPI-deficient host cell cultured in rich medium. Preferred promoters useful in yeast expression vectors include promoters from yeast glycolytic genes (Hitzeman et al., J. Biol. Chem. 255:12073-12080, 1980; Alber and Kawasaki, J. Mol. Appl. Genet. 1:419-434, 1982; Kawasaki, U.S. Patent No. 4,599,311) or alcohol dehydrogenase genes (Young et al., in Genetic Engineering of Microorganisms for Chemicals, Hollaender et al. (eds.), p. 335, Plenum, New York, 1982; Ammerer, Meth. Enzymol. 101:192-201, 1983; Russell et al., Nature 304: 652-654, 1983). In this regard, particularly preferred promoters are the TPI1 promoter, the GAL10 promoter, and the ADH2-4$^c$ promoter. In addition, it is preferable to include a transcriptional termination signal, such as the TPI1 terminator, within the expression vector. To facilitate purification of polypeptides produced in a yeast transformant, a signal sequence, preferably from a yeast gene encoding a secreted protein, may be joined to the coding sequence for the protein of interest. A particularly preferred signal sequence is the pre-pro region of the MFα1 gene (Kurjan and Herskowitz, Cell 30:933-943, 1982; Kurjan et al., U.S. Patent 4,546,082). Another preferred signal peptide is a 19 residue signal peptide designed according to the rules of von Heijne (Eur. J. Biochem. 133:17-21, 1983; J. Mol. Biol. 184:99-105, 1985; Nucleic Acids Res. 14:4683-4690, 1986).

Higher eukaryotic cells may also serve as suitable host cells within the present invention, with cultured mammalian cells preferred. Expression vectors for use in mammalian cells will comprise a promoter capable of directing the transcription of a foreign gene introduced into a mammalian cell. Particularly preferred promoters are the mouse metallothionein-1 (MT-1) promoter (Palmiter et al., Science 222:809-814, 1983), or the major late promoter of adenovirus 2. Also included in such expression vectors is a polyadenylation signal, located downstream of the DNA sequence insertion site. The polyadenylation signal may be that of the cloned gene, or may be derived from a heterologous gene. Other sequences, such as enhancers and RNA splicing signals, may also be included. Expression vector construction is within the level of ordinary skill in the art.

Cloned DNA sequences may then be introduced into cultured mammalian cells by, for example, calcium phosphate-mediated transfection (Wigler et al., Cell 14:725, 1978; Coraro and Pearson, Somat. Cell Genet. 7:603, 1981; Graham and Van der Eb, Virol. 52:456, 1973). A precipitate of DNA and calcium phosphate is formed, and this precipitate is applied to the mammalian cells. Some of the cells take up the foreign DNA and maintain it inside the host cell for several days. A small fraction of these cells (typically about one in $10^4$) integrate the heterologous DNA into the genome. In order to identify these integrants, a gene that confers a selectable phenotype (a selectable marker) is generally introduced into the cells along with the gene of interest. Preferred selectable markers include genes that confer resistance to drugs, such as neomycin, hygromycin, and methotrexate. Selectable markers may be introduced into the cell on a separate expression vector at the same time as the gene of interest, or they may be introduced on the same expression vector. Alternative transfection techniques such as electroporation (Neumann et al., EMBO J. 1:841-845, 1982) may also be used.

The copy number of the integrated gene sequence may be increased through amplification by drug selection on the selectable marker. The drug concentration is increased in a stepwise manner, with selection of resistant cells at each step. By selecting for increased copy number of cloned sequences, expression levels may be substantially elevated.

The selected host cells are grown in an appropriate culture medium and the recombinant Factor XIII may be isolated according to standard procedures. Briefly, Factor XIII is isolated by DEAE-cellulose chromatography, followed by fractionation on a Sepharose-6B column. The column is eluted with a suitable buffer, such as 50 mM Tris-HCl, pH 7.5, containing 1 mM EDTA. The active peak is concentrated by precipitation with 40% ammonium sulfate. Alternatively, procedures such as immunoaffinity chromatography or HPLC may be employed.

One application of the proteins produced by the methods described herein is as pharmaceutical compositions. The proteins can be purified and formulated in a convenient manner, resulting in a suitable concentration in relation to the particular host to be treated. Physiologically acceptable carriers or diluents such as sterile water, saline, and buffered saline can also be employed. Such a pharmaceutical composition can be administered in a variety of different ways. The proteins may also be used in formulating tissue sealants.

The following examples are offered by way of illustration and not by way of limitation.

EXAMPLES

Unless otherwise indicated, standard molecular biological methods were used.

Restriction endonucleases, exonuclease BAL-31, and $T_4$ DNA ligase were obtained from Bethesda Research Laboratories or New England Biolabs. The Klenow fragment of Escherichia coli DNA polymerase, bacterial alkaline phosphatase, ATP, deoxynucleotides, dideoxynucleotide triphosphates, M13mp10, M13mp11, M13mp18, M13mp19, pUC9, and pUC19 were obtained from Bethesda Research Laboratories. A human placenta cDNA library was obtained from Clontech Lab., Inc. (Palo Alto, Calif). $Na^{125}I$ and $^{32}P$-labeled nucleotides and $[\alpha^{35}S]dATP$ were obtained from New England Nuclear or Amersham. Normal human plasma was provided by the Pacific Northwest Red Cross Blood Service, Portland, Oregon.

Factor XIII was purified from human plasma according to the method of C.G. Curtis and L. Lorand, Meth. Enzymol. 45:177-191, 1976. It was converted to Factor XIIIa in the presence of thrombin, and the thrombin was then inactivated by hirudin. The a′ and b subunits were fractionated by gel filtration on a Bio-Gel A-5m column according to the method of Chung et al., ibid.

Polyclonal antibodies against the a′ subunit and the b subunit of Factor XIII were raised in rabbits, and the immunoglobulin fractions were purified by ammonium sulfate fractionation, DEAE-Sephadex column chromatography, and affinity chromatography employing an immobilized antigen-Sepharose column.

Oligonucleotides were synthesized on an Applied Biosystems (Foster City, Calif.) Model 380A synthesizer and purified on polyacrylamide gels.

EXAMPLE I

Cloning of a cDNA Encoding the a Subunit of Human Factor XIII

A. Screening for cDNA Encoding the a Subunit of Human Factor XIII

A λgt11 expression library containing cDNAs prepared from human placenta mRNA was screened for the a subunit of human Factor XIII. An $^{125}I$-labeled affinity-purified rabbit antibody (specific activity = 6 X $10^6$ cpm/ug) was used to screen filters containing phage plated at a density of 1.5 X $10^5$ plaques per 150 mm plate. Six positive clones were isolated by screening approximately 3 X $10^6$ phage, and each positive phage was plaque-purified.

Plaque-purified clones were then screened with the following $^{32}$P-labeled oligonucleotide probe: $^{5'}$CTC CAC GGT GGG CAG GTC GTC CTC G$^{3'}$. This probe codes for the amino acid sequence of Ala-Glu-Asp-Asp-Leu-Pro-Thr-Val-Glu that is present in the activation peptide of the a subunit (Takagi and Doolittle, ibid.). The nucleotide sequence for the probe was selected by employing the most common codon usage for amino acids for a number of different human proteins (Chen and Barker, Trends in Genetics 1:221-223, 1985). The oligonucleotide was labeled with $^{32}$P to a specific activity of 1.1 X 10$^8$ cpm/ug.

B. DNA Sequencing of cDNA Inserts

Phage DNA was prepared from positive clones by the liquid culture lysis method (T.J. Silhavy et al., in Experiments with Gene Fusions, CSH Laboratory, N.Y., pp. 140-141, 1984), followed by centrifugation and banding on a cesium chloride step gradient. cDNA inserts were isolated by digestion of the phage DNA with Eco RI endonuclease and the 5$^{'}$ and 3$^{'}$ ends of each insert were sequenced. One of the clones with a large cDNA insert (λHFXIIIa3.77, ATCC No. 40261) was selected for further sequence analysis. This insert contained three internal Eco RI sites, giving rise to four cDNA fragments upon digestion of the phage DNA with Eco RI. These fragments and several additional restriction fragments were subcloned into plasmid pUC9 or pUC19. Additional restriction fragments from other cDNA inserts were subcloned into M13mp10, M13mp11, M13mp18, or M13mp19 in order to obtain overlapping sequences. The cDNA inserts were then sequenced by the dideoxy method (F. Sanger et al., Proc. Natl. Acad. Sci. USA 74:5463-5467, 1977) using [$α^{35}$S]dATP and buffer gradient gels (M. D. Biggin et al., Proc. Natl. Acad. Sci. USA 80:3963-3965, 1983). Digestions with nuclease BAL-31 were performed to generate five additional fragments that provided overlapping sequence with the Eco RI restriction fragments.

The sequence of 3831 base pairs from these overlapping clones and the predicted amino acid sequence are shown in Figure 1. This DNA sequence codes for the entire amino acid sequence of the mature a subunit of human Factor XIII that circulates in blood. The a subunit is composed of 731 amino acids, starting with an amino-terminal sequence of Ser-Glu-Thr-Ser. This amino-terminal sequence was reported earlier by Takagi and Doolittle, ibid. The carboxyl-terminal Met (nucleotides 2281-2283) is followed by a stop codon (TGA), 1535 base pairs of noncoding sequence, and a potential polyadenylation or processing signal of AATAAA. The polyadenylation sequence was located 14 nucleotides upstream from the poly(A) tail of 10 nucleotides. The poly(A) tail was present only in a second cDNA clone, designated λHFXIIIa3.82.

The cDNA insert in λHFXIIIa3.77 also codes for 30 amino acid residues that appear to encode an in-frame sequence. Cleavage of the bond between Met and Ser by a processing protease to yield mature Factor XIII would require an additional processing protease(s) to yield the mature protein with an amino-terminal Ser. This would be followed by an acetylation reaction, leading to the formation of acetyl-serine at the amino-terminal end of the mature protein (Takagi and Doolittle, ibid.; S. Nakamura et al., J. Biochem. 78:1247- 1266, 1975).

A difference in the nucleotide sequence for the a subunit of Factor XIII was found at three positions when a comparison of the cDNA inserts was made in regions where overlapping sequences were obtained. Nucleotides 2038, 2041, and 2727 contained A, C, and T, respectively, in λHFXIIIa3.77, (Figure 2), while λHFXIIIa3.82 contained C, G, and A in the same positions. These differences results in a change in two amino acids (Ile 650 and Gln 651 to Val and Glu), and could represent a polymorphism that contributes to the microheterogeneity in the a subunit of Factor XIII (P.G. Board and M. Coggan, Hum. Genet. 59:135-136, 1981).

C. Amino Acid Sequencing

Amino acid sequence analyses were also performed on cyanogen bromide fragments of the a subunit of human Factor XIII. Briefly, 10 milligrams of Factor XIII were dialyzed overnight against 5% HCOOH, and the dialysate was adjusted to pH 4.0 with ammonium hydroxide. The precipitate that was enriched with the a subunit (83% a and 17% a subunits) was S-carboxymethylated and digested with cyanogen bromide. The resulting fragments were separated by gel filtration on a Sephadex G-50 superfine column using 5% HCOOH and further purified on a Waters HPLC system using an Ultrapore C3 reverse-phase column (Altex). The gradient employed consisted of 0.1% trifluoroacetic acid as a mobile phase and 0.8% trifluoroacetic acid in 80% acetonitrile as a mobile phase modifier. The column was run at a flow rate of 1.5 ml/min, and the eluant was monitored by absorbance at 214 nm.

Eleven of the eighteen cyanogen bromide fragments expected from the cDNA were isolated. Peptides arising from the b subunit (A. Ichinose et al., Biochem. 25:4633-4638, 1986) were readily identified and discarded from the remaining peptides originating from the a subunit. Sequence analysis of each of the purified cyanogen bromide fragments was performed with a Beckman automated sequenator, model 890C, by the method of P. Edman and G. Begg, Eur. J. Biochem. 1:80-91, 1967. PTH-amino acids were identified by two complementary reverse-phase column systems (L.H. Ericsson et al., in Solid Phase Methods in Protein Sequence Analysis, A. Previero and M.A. Coletti-Previero (eds.), pp. 13-142, 1977; J.L. Glajch et al., J. Chromatogr. 318:23-29, 1985). A total of 363 residues were unequivocally identified (residues overlined in Figure 1). These amino acid sequences were in complete agreement with those predicted by the cDNA.

The protein sequence predicted from the cDNA includes six potential Asn-linked glycosylation sites with a sequence of Asn-X-Ser or Asn-X-Thr. These Asn residues are located at positions 17, 46, 541, 556, 613, and 686. Two of these Asn residues have little or no carbohydrate, since Asn was readily identified in position 613 and 686 by the amino acid sequence analysis. Furthermore, carbohydrate was not reported in Asn 17 by Takagi and Doolittle (ibid.). Since the a subunit of Factor XIII contains only 1.5% carbohydrate, it is also possible that positions 46, 541, and 556 may contain little or no carbohydrate. A partial glycosylation of some of these Asn residues, however, could contribute to the microheterogeneity of the a subunit of human Factor XIII (Board and Coggan, ibid.).

The a subunit of Factor XIII consists of 731 amino acid residues with the following composition: $Ala_{37}$, $Arg_{45}$, $Asn_{40}$, $Asp_{47}$, $1/2Cys_9$, $Gln_{27}$, $Glu_{48}$, $Gly_{50}$, $His_{14}$, $Ile_{39}$, $Leu_{48}$, $Lys_{38}$, $Met_{19}$, $Phe_{32}$, $Pro_{33}$, $Ser_{45}$, acetyl $Ser_1$, $Thr_{45}$, $Trp_{15}$, $Tyr_{29}$, $Val_{70}$. The molecular weight of the polypeptide portion of the molecule was calculated to be 80,488. The addition of 1.5% carbohydrate gives a molecular weight of approximately 81,700 for each of the a subunits of human Factor XIII. This is in good agreement with the value of 75,000 estimated by SDS-polyacrylamide gel electrophoresis (Chung et al., ibid.).

Activation of Factor XIII by thrombin is due to the cleavage of an activation peptide from the amino-terminus of each of the a subunits of the molecule (Schwartz et al., ibid.). The amino-terminal sequence of the a subunit of Factor XIII deduced from all six cDNA clones was the same as that reported by Takagi and Doolittle (ibid.) except for an additional Val at residue 34. Accordingly, the cDNA data predict an activation peptide of 37 rather than 36 amino acids. The a subunit of bovine Factor XIII contains a Leu residue in position 34 instead of a Val, and the activation peptide is also 37 amino acids in length (Nakamura et al., ibid.).

The carboxyl-terminal residue of the a subunit of Factor XIII was identified as Met, which is the same as that of the bovine molecule. Both the amino- and carboxyl-terminal sequences of the a subunit of Factor XIII, however, are totally different from those of tissue transglutaminase (J.M. Connellan et al., J. Biol. Chem. 246:1093-1098, 1972).

The amino acid sequences at the active sites of plasma Factor XIII and tissue transglutaminase have been identified as Gly-Gln-Cys-Trp and Tyr-Gly-Gln-Cys-Trp, respectively (Cooke, ibid.; J.E. Folk and P.W. Cole, J. Biol. Chem. 241:3238-3240, 1966). The present results from cDNA and amino acid sequence analyses indicate that the active site sequence of Tyr-Gly-Gln-Cys-Trp starts at residue Tyr 311.

DNA sequences were analyzed by a computer program of Textco (W. Lebanon, N.H.) using an Apple MacIntosh computer. A computer-assisted analysis using a Dayhoff program (M.O. Dayhoff et al., Meth. Enzymol. 91:524-545, 1983) revealed that the amino acid sequence for the a subunit of human Factor XIII is unique, and little significant homology was found with any other protein, other than the active site of transglutaminase and minor sequence homology with the γ subunit of acetylcholine receptor.

EXAMPLE II

Cloning of a cDNA Encoding the b Subunit of Human Factor XIII

A. Screening a cDNA library

A λgt11 expression library containing cDNAs prepared from human liver mRNA was screened for the b subunit of human Factor XIII employing a $^{125}$I-labled, affinity-purified rabbit antibody. The purified antibody was labeled with $Na^{125}I$ to a specific activity of 4 X $10^6$ cpm/ug, and was used to screen filters containing phage plated at a density of 1.5 X $10^5$ plaques per 150 mm plate. Nine positive clones were isolated by screening 2 X $10^6$ phage, and each was plaque-purified.

B. DNA Sequencing of cDNA Inserts

Phage DNA was prepared from positive clones by the liquid culture lysis method (Silhavy et al., ibid.), followed by centrifugation and banding on a cesium chloride step gradient. The clone with the largest cDNA insert (approximately 2.2 kilobases) was designated λHFXIIIb2.2 and was selected for further study. The phage DNA from λHFXIIIb2.2 was cut with Eco RI to isolate the 2.2. kb cDNA insert. This fragment was subcloned into plasmid pUC9 which had been linearized by digestion with Eco RI to construct plasmid pUC9b2.2 (ATCC No. 40260). Appropriate restriction fragments from the insert were then subcloned into M13mp10 or M13mp18 for sequencing by the dideoxy method (Sanger et al., ibid.) using $[\alpha^{35}S]$dATP and buffer gradient gels (Biggin et al., ibid.). Controlled digestions with nuclease BAL-31 were performed to generate suitable fragments, which provided overlapping sequences with the restriction fragments. All sequence determinations were performed on both stands of DNA at least three times.

The cDNA insert was found to be composed of 2180 base pairs coding for the entire amino acid sequence for the b subunit of Factor XIII that circulates in blood (Figure 2). The mature b subunit is composed of 641 amino acids, starting with amino-terminal Glu (nucleotides 56-58). The amino-terminal sequence of Glu-Glx-Lys-Pro for the b subunit of human Factor XIII was established earlier by Takagi and Doolittle (ibid.) and was extended by the present invention. The carboxy-terminal Thr (nucleotides 1979-1981) is followed by a stop codon (TAG), 187 base pairs of noncoding sequence, and a poly(A) tail of 9 base pairs. The polyadenylation or processing signal of AATAAA was identified 19 nucleotides upstream from the poly(A) tail.

The cDNA clone also codes for 19 amino acid residues that constitute a portion of a leader peptide. The partial leader sequence of 19 amino acids includes a typical hydrophobic core and an Ala residue at position -1 and Leu at postion -3. These residues are consistent with the "-1 and -3 rule," in which the -1 position is occupied predominantly by Ala in signal sequences (D. Perlman and H.O. Halvorson, J. Molec. Biol. 167:391-409, 1983; G. von Heijne, J. Molec. Biol. 173:243-251, 1984). Amino acid sequence analysis of the intact position was then carried out in a Beckman sequenator and 19 amino acids were identified. This extended the amino terminal sequence originally reported by Takagi and Doolittle, ibid.

C. Amino Acid Sequencing

Amino acid sequence analyses were also performed on cyanogen bromide fragments of the b subunit of human Factor XIII. Factor XIII was purified from human plasma according to the method of Curtis and Lorand, ibid. After incubation with thrombin, the b subunit of Factor XIII was separated from the a subunit by gel filtration. The purified b subunit (10 mg) was then S-pyridylethylated (M. Friedman et al., J. Biol. Chem. 245:3868-3871, 1970) and digested with cyanogen bromide. The resulting fragments were separated and purified according to the procedure described in Example I.C. above.

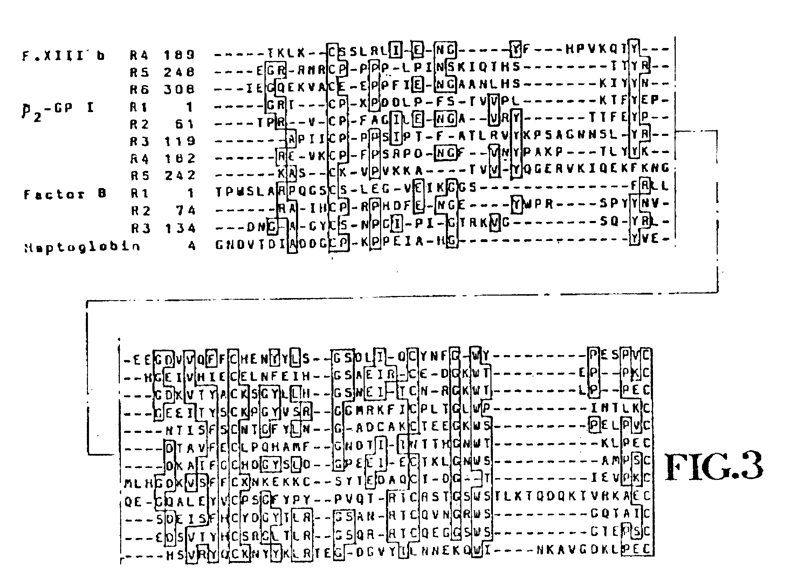

Nine of the ten cyanogen bromide fragments expected from the amino acid sequence obtained from the cDNA was isolated, and corresponded to cyanogen bromide fragments 1-4 and 6-10, as numbered from the amino-terminal to the carboxyl-terminal end of the protein (Figure 3). Each of these fragments was then subjected to amino acid sequence analysis, as described in Example I.C., and a total of 299 residues were unequivocally identified. These amino acid sequences were in complete agreement with those predicted by the cDNA and the amino terminal sequence analysis of the intact protein.

The b subunit of Factor XIII consists of 641 amino acid residues and has the following composition: $Ala_{17}$, $Arg_{26}$, $Asn_{30}$, $Asp_{22}$, $1/2Cys_{40}$, $Gln_{20}$, $Glu_{60}$, $Gly_{48}$, $His_{18}$, $Ile_{29}$, $Leu_{47}$, $Lys_{44}$, $Met_9$, $Phe_{20}$, $Pro_{41}$, $Ser_{45}$, $Thr_{44}$, $Trp_{10}$, $Tyr_{42}$, $Val_{29}$. The molecular weight of the polypeptide portion was calculated to be 69,973. The addition of 8.5% carbohydrate gives a molecular weight of approximately 76,500 for each of the b subunits of human Factor XIII. This is in good agreement with the value of 80,000 estimated by SDS polyacrylamide gel electrophoresis (Schwartz et al., ibid.; Chung et al., ibid.).

The protein sequence predicted from the cDNA includes two potential Asn-linked glycosylation sites with the sequence of Asn-Tyr-Ser and Asn-Gly-Ser, starting with amino acid residues 142 and 525, respectively. In addition, a third potential carbohydrate attachment site is present in the sequence of Asn-Arg-Cys, starting at residue 252. The attachment of carbohydrate chains to Asn residues in a sequence of Asn-X-Cys was first reported in bovine protein C (J. Stenflo and P. Fernlund, J. Biol. Chem. 257:12180-12185, 1982) and later in human von Willebrand factor (K. Titani et al., Biochem. 25:3171-3184, 1986). The differential glycosylation of those Asn residues may be in part responsible for the microheterogeneity of the b subunit of human Factor XIII (C.G. Curtis et al., Biochem. 13:3774-3780, 1974; P.G. Board, Am. J. Hum. Genet. 32:348-353, 1980). The 20 potential disulfide bonds identified by the cDNA coding for the b subunit of Factor XIII are in reasonably good agreement with the 16 to 17 disulfide bonds reported earlier (Chung et al., ibid.). The b subunit of Factor XIII lacks free -SH groups.

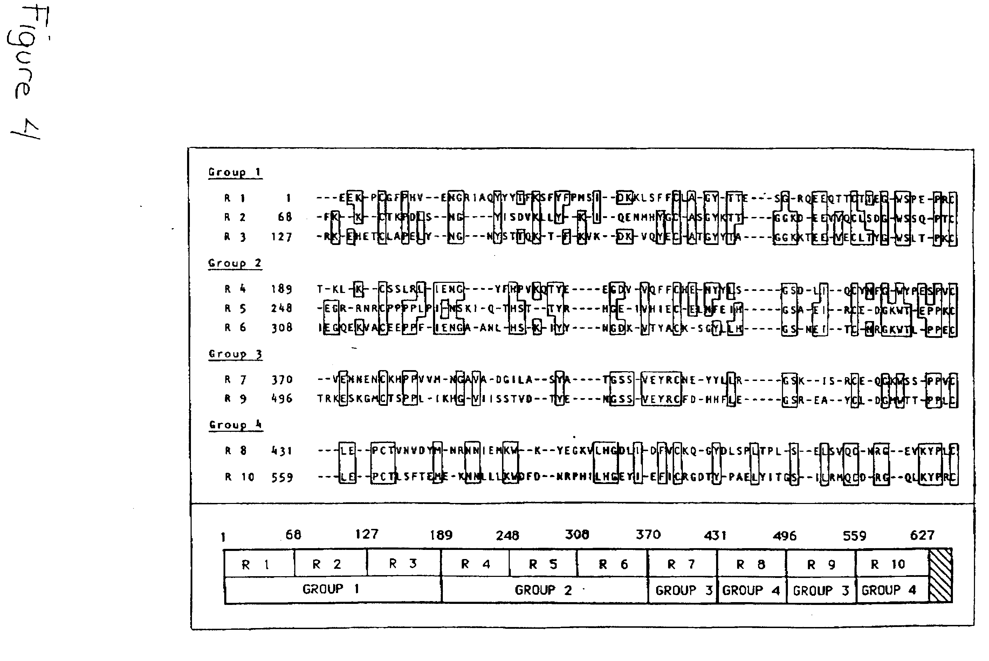

The amino acid sequence of the b subunit of Factor XIII shows evidence of considerable internal gene duplication, involving ten repetitive sequences of approximately 60 amino acids. These repetitive sequences were subclassified into four distinct groups (Figure 4). The identities within groups 1, 2, 3, and 4 were 34-42%, 34-42%, 38%, and 41%, respectively. Although the identities among the four groups of repeats are obviously less than each internal identity, high alignment scores employing the Dayhoff program (Dayhoff et al., ibid.) indicate that these four groups have diverged from one prototype. For instance, the alignment scores were 7.7 between repeats 3 and 4, 12.1 between repeats 5 and 7, and 3.4 between repeats 6 and 8. The ten repeats (1-10) are aligned consecutively throughout 98% of the molecule and include amino acid residues 1 to 626. A short sequence of 15 amino acids (residues 627 to 641) following the last repeat at the carboxyl end of the molecule was not homologous with repeats 1-10.

Computer-assisted analysis using a Dayhoff program revealed that the repeated sequences in the b subunit of Factor XIII are members of a family of repeats that are very similar to three repeated segments present in the Ba chain of factor B of human complement (J.E. Mole et al., J. Biol. Chem. 259:3407-3412, 1984), five repeated segments in human $\beta_2$-glycoprotein I (J. Lozier et al., Proc. Natl. Acad. Sci. USA 81:3640-3644, 1984) [a protein identical to activated protein C binding protein (W.M. Canfield and W. Kisiel, J. Clin. Invest. 70:1260-1272, 1982)] and human haptoglobin $\alpha^1$ chain (A. Kurosky et al., Proc. Natl. Acad. Sci. USA 77:3388-3392, 1980). This sequence homology is shown in Figure 3. The fourth segment of the $\beta_2$-glycoprotein I, the second segment of complement factor Ba, and human haptoglobin $\alpha^1$ chain show the highest alignment scores (11, 8.0, and 4.2, respectively) with repeat 6 of the b subunit of human Factor XIII. These data indicate that these four proteins share a common ancestry and have probably resulted from exon shuffling during evolution.

The location of five disulfide bonds in the five homologous segments in $\beta_2$-glycoprotein I has been established and shown to occur between the first and third and the second and fourth Cys residues in each segment (Lozier et al., ibid.). Thus it seems probable that a similar pairing occurs with the disulfide bonds in the ten repeats in the b subunit of Factor XIII.

EXAMPLE III

Cloning and Subcloning of Promoters

A. Construction of pMVR1

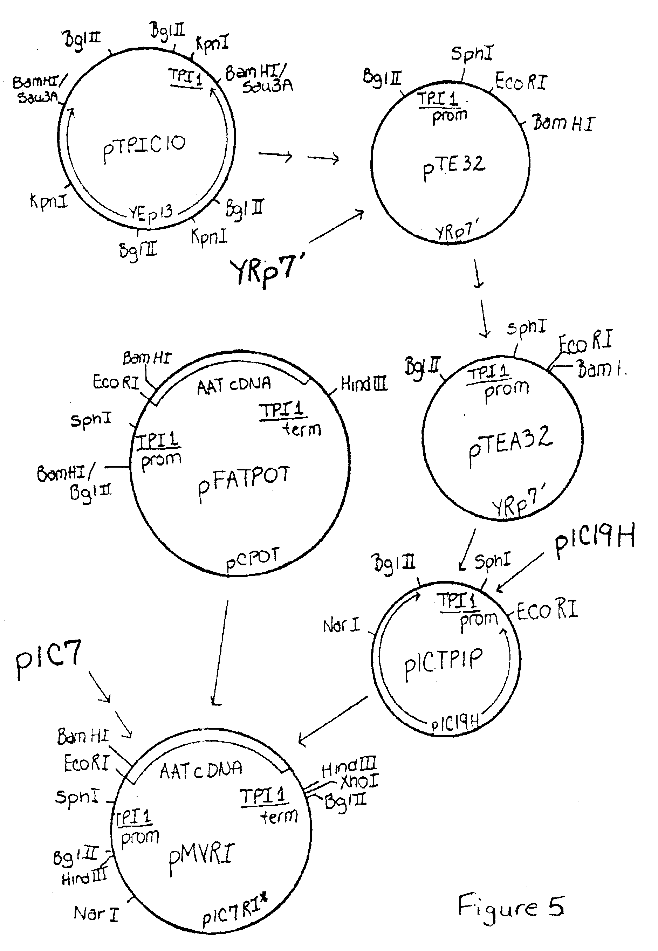

Plasmid pMVR1, used as the source of the TPI1 promoter in subsequent vector constructions, comprises the TPI1 promoter, an alpha-1-antitrypsin (AAT) cDNA and the TPI1 terminator in the vector pIC7RI*. Plasmid pMVR1 was constructed as follows (Figure 5). Plasmid pIC7 (Marsh et al., Gene 32: 481-486, 1984) was digested with Eco RI, the fragment ends blunted with DNA polymerase 1 (Klenow fragment, and the linear DNA recircularized using T4 DNA ligase. The resulting plasmid was used to transform E. coli strain RRI. Plasmid DNA was prepared from the transformants and screened for the loss of the Eco RI site. A plasmid having the correct restriction pattern was designated pIC7RI*. The TPI1 promoter fragment was obtained from plasmid pTPIC10 (Alber and Kawasaki, ibid.). This plasmid was cut at the unique Kpn I site within the TPI1 gene and the TPI1 coding region was removed by treatment with nuclease BAL-31. Kinased Eco RI linkers (GGAATTCC) were added to the fragment which was then digested with Bgl II and Eco RI to yield a 0.9 kb TPI1 promoter fragment. This fragment was joined to plasmid YRp7' (Stinchcomb et al., Nature 282:39-43, 1979) which had been cut with Bgl II and Eco RI. The resultant plasmid, pTE32, was cleaved with Eco RI and Bam HI to remove a portion of the tetracycline resistance gene. The linearized plasmid was then recircularized by the addition of a kinased Eco RI-Bam HI oligonucleotide adapter (5' AAT TCA TGG AG 3' and 5' GAT CCT CCA TG 3'). The resultant plasmid, pTEA32, was digested with Bgl II and Eco RI to isolate the 900 bp TPI1 promoter fragment. This fragment was joined with pIC19H (Marsh et al., ibid.) which had been linearized by digestion with Bgl II and Eco RI. The resultant plasmid was designated pICTPI. Plasmid pFATPOT (deposited as a S. cerevisiae transformant in strain E18, ATCC No. 20699) was digested with Sph I and Hind III to isolate the 1750 bp fragment comprising the partial TPI1 promoter, a cDNA encoding human alpha-1-antitrypsin, and the TPI1 terminator. Plasmid pICTPI was digested with Nar I and Sph I to isolate the 1.1 kb fragment comprising the partial TPI1 promoter and lacZ' coding sequence. Plasmid pIC7RI* was digested with Hind III and Nar I to isolate the 2.5 kb vector fragment. The pIC7RI* fragment, the partial TPI1 promoter fragment derived from plasmid pICTPI and the 1.75 kb fragment derived from plasmid pFATPOT were joined in a three part ligation to produce plasmid pMVR1.

B. Construction of pTRK4c

The ADH2-4$^c$ promoter was derived from plasmids pBR322-ADR2-BSa (V.M. Williamson et al., Cell 23:605-614, 1981) and YRp7-ADR3-4$^c$ (D.W. Russell et al., ibid.). An Eco RI site was placed just 3' to the translation start codon of the ADH2 promotor derived from plasmid pBR322-ADR2-BSa by in vitro mutagenesis. Following the mutagenesis, 5' flanking sequences which confer the ADH2-4$^c$ phenotype were used to replace the analogous sequences of the ADH2 promoter. The 2.2 kb Bam HI fragment from pBR322-ADR2-BSa containing the ADH2 structural gene and 5' flanking sequences was ligated with M13mp19 linearized with Bam HI. The orientation of the insert was determined by restriction analysis. Single-stranded template DNA was made from the resultant phage clone. Site-specific in vitro mutagenesis (M.J. Zoller and M. Smith, DNA 3:479-488, 1984) was carried out on the template using oligonucleotide ZC237 (Table 1) to loop out the structural portion of the ADH2 gene, fusing the 5' flanking sequence, including the translation start signal, with the Eco RI site of the M13mp19 polylinker. The replicative form of the mutagenized phage was made and cut with Bam HI and Eco RI to isolate the 1.2 kb promoter fragment. This fragment was ligated into pUC13 which had been linearized by digestion with Bam HI and Eco RI to generate plasmid p237WT. To change the p237WT promoter to the "promoter up" mutant ADH2-4$^c$, a 1.1 kb Bam HI-Sph I partial promoter fragment from YRp7-ADR3-4$^c$ was subcloned into the vector fragment of p237WT cut with Bam HI and Sph I. The resultant plasmid was designated p237-4$^c$.

## TABLE 1

ZC87    5'TCC CAG TCA CGA CGT3'

ZC212   5'GAC CTG CAG GAT CCA TGC AGC GCC TGA ACA TGA TCA TGG3'

ZC213   5'GAG CCC TGG TGA TTC TGC CAT GAT CAT GTT CAC GCG CTG3'

ZC235   5'GAT CCA TCC AGC GC3'

ZC237   5'GCC AGT GAA TTC CAT TGT GTA TTA3'

ZC410   5'CGT ATT ACA GAA TTC CCC GG3'

ZC862   5'CGA ATC TTT TGA CCT CAG AAA CAC C3'

ZC1019  5'ACC CAA GGA TCT CTT GTC CAA AGA AAC ACC TCC TTC3'

ZC1056  5'ATT TAG ATC TGC A3'

ZC1057  5'GAT CT3'

ZC1059  5'TCA AAG AAC CCA AGG AAG CTT CAG CCT CTC TTT TAC CCA AAG AAA C3'

ZC1113  5'CGA CCT TCC ATG TGA TAA CTC GAG AAC CTG AGA TGA AC3'

```
ZC1206      5'GGA CCT TGT AAA GTG AGA AGC TTC AGA AAC TTC CAG
            GA3'

ZC1209      5'GAT GTT CTT GCA AGC TTT CTT GTT CTT GTT GGC TGG
            TTT CGC AGG TAC CGA3'

ZC1210      5'AGC TTC GGT ACC TGG AGC GAA ACC AGC CAA CAA GAA
            CAA GAA AGC TTG CAA CAA CAT CTC CA3'
```

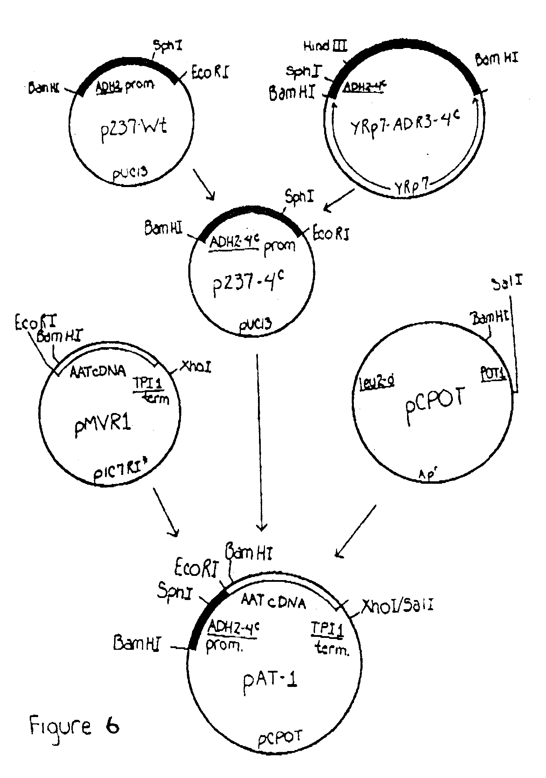

Referring to Figure 6, the ADH2 promoter from p237WT was modified to create a "universal" ADH2 promoter. The promoter was first subcloned into plasmid pCPOT (deposited with ATCC as an E. coli strain HB101 transformant, ATCC No. 39685), which comprises the entire 2 micron plasmid DNA, the leu2-d gene, pBR322 vector sequences and the Schizosaccharomyces pombe POT1 gene. Plasmid pCPOT was digested to completion with Bam HI and Sal I to isolate the approximately 10 kb linear vector fragment. Plasmid pMVR1 (Example III.A.) was cut with Eco RI and Xho I to isolate 1.5 kb AAT cDNA-TPI1 terminator fragment. The 1.2 kb ADH2 promoter fragment was isolated from plasmid p237WT as a Bam HI-Eco RI fragment and ligated with the 1.5 kb AAT cDNA-TPI1 terminator fragment and the linearized pCPOT in a three-part ligation to yield the plasmid designated pAT-1.

The ADH2 promoter present in plasmid pAT-1 was then modified to create a "universal" promoter by removing the translation start codon and fusing the promoter to an Eco RI site. Plasmid pAT-1 was digested with Sph I and Bam HI to isolate the 190 bp fragment comprising the ADH2 promoter from the Sph I site through the Bam HI site of the AAT cDNA. This fragment was ligated into M13mp18 which had been linearized by digestion with Bam HI and Sph I. A positive clone was confirmed by restriction analysis. Template DNA was made from the positive clone. Oligonucleotide ZC410 (Table 1) was designed to replace the ADH2 translation start signal and pUC18 polylinker sequences with a single Eco RI site fused to the M13mp18 polylinker at the Sma I site. The template was subjected to in vitro mutagenesis using the two-primer method (Zoller and Smith, ibid., 1984) using oligonucleotide ZC410 and ZC87 (Table 1). Positive clones were confirmed by dideoxy sequencing through the fusion point. For ease of manipulation, the 175 bp Sph I-Eco RI mutagenized promoter fragment was ligated into pUC19 which had been linearized with Sph I and Eco RI. The resultant plasmid, designated p410ES, comprises the 3′ portion of a "universal" ADH2 promoter.

A "universal" ADH2-4[c] promoter was constructed using the mutagenized ADH2 promoter fragment from p410ES and the ADH2-4[c] promoter fragment from p237-4[c]. Plasmid p410ES was digested with Sph I and Eco RI to isolate the 175 bp partial ADH2 promoter fragment. Plasmid p237-4[c] was cut with Bam HI and Sph I to isolate the 1 kb partial ADH2-4[c] promoter fragment. The ADH2-4[c] promoter was reconstructed in a three-part ligation with the Bam HI-Sph I promoter fragment from p237-4[c], the Sph I-Eco RI promoter fragment from p410ES, and pUC13 which had been linearized by digestion with Bam HI and Eco RI. The resultant plasmid, p410-4[c], comprised a "universal" ADH2-4[c] promoter.

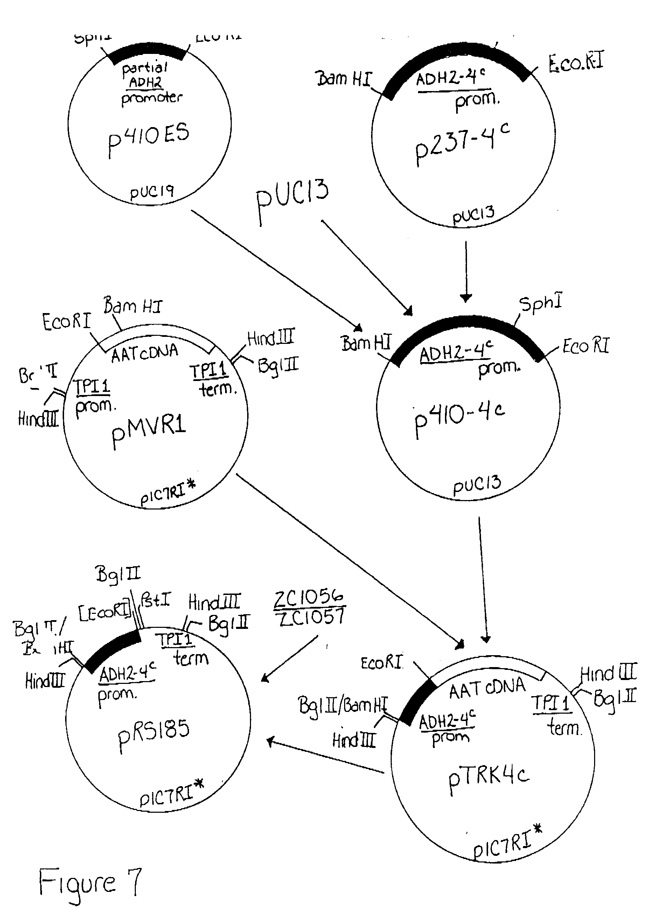

As illustrated in Figure 7, the "universal" promoter from plasmid p410-4[c] was used to replace the TPI1 promoter present in plasmid pMVR1 (Example III.A.). Plasmid p410-4[c] was cut with Bam HI and Eco RI to isolate the 1.2 kb ADH2-4[c] promoter fragment. Plasmid pMVR1 was cut with Bam HI and Eco RI to isolate the 1.2 kb ADH2-4[c] promoter fragment. Plasmid pMVR1 was digested to completion with Eco RI and partially digested with Bgl II to isolate the 4.2 kb AAT-TPI1 terminator-vector fragment. These two fragments were ligated to form the plasmid designated pTRK4c.

EXAMPLE IV

Construction of the Vector pEAS102

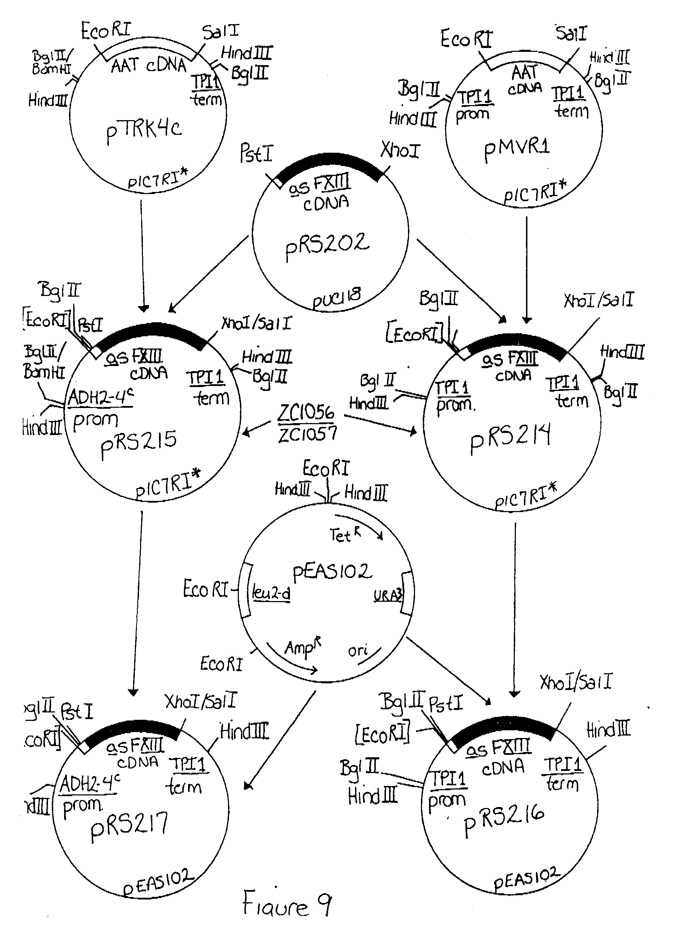

Plasmid pEAS102, comprising portions of the vectors YIp5 and pJDB207, was constructed as follows. Plasmid pJDB207 (J.D. Beggs, Proceedings of Alfred Benzon Symposium 16:383-389, "Molecular Genetics in Yeast," Copenhagen, Denmark, 1981), a derivative of pJDB219 (J.D. Beggs, Nature 275:104-108, 1978), was digested with Bam HI and Pst I to isolate the 4.4 kb fragment comprising the leu2-d gene and 2 micron DNA and pBR322 sequences. Plasmid YIp5 (Struhl et al., ibid.) was subjected to partial digestion with Pst I and complete digestion with Bam HI to isolate 4.3 kb fragment comprising the URA3 gene and pBR322 sequences. These two fragments were ligated and the resultant plasmid was designated pEAS102 (illustrated in Figure 9).


EXAMPLE V

Expression of the Factor XIII a Subunit in Yeast


A. Construction of pRS202


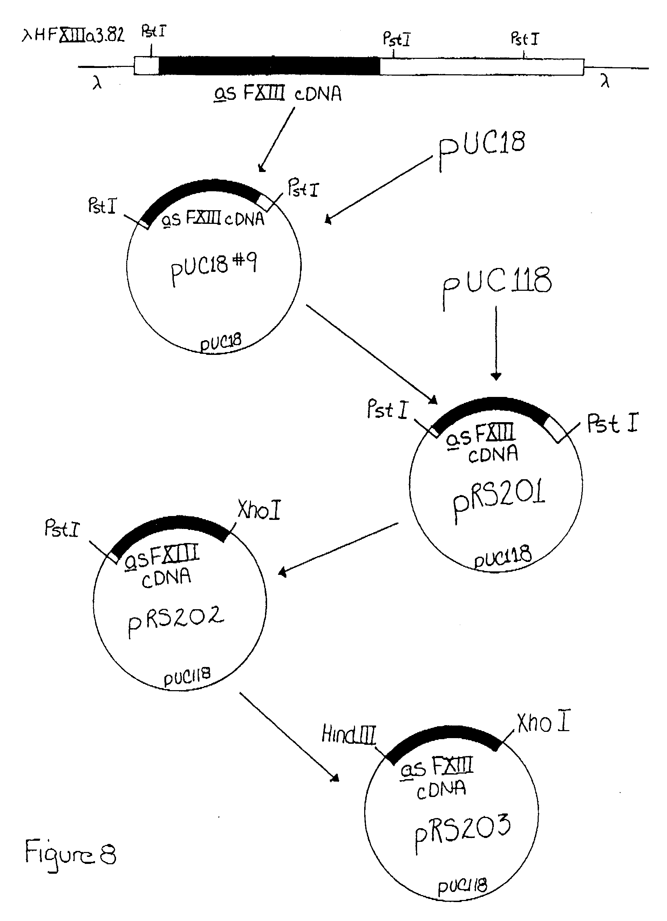

The Factor XIII a subunit (asFXIII) cDNA was modified by in vitro mutagenesis to replace the 3′ noncoding region with an Xho I site (Figure 8). For ease of manipulation, the 3.8 kb asFXIII cDNA insert contained in phage clone λHFXIIIa3.82 was subcloned into pUC18. The phage clone λHFXIIIa3.82 was digested to completion with Pst I to isolate the 2.3 kb fragment comprising the asFXIII cDNA. This fragment was ligated with pUC18 which had been linearized by digestion with Pst I. The resultant plasmid, pUC18 #9, was determined to have the 2.3 kb Pst I insert in the anti-sense orientation. The asFXIII cDNA insert present in pUC18 #9 comprised 19 bp 5′ to the translation start, the asFXIII coding region and 120 bp 3′ to the translation stop.

The asFXIII cDNA insert was then isolated and subcloned into the vector pUC118 (obtained from J. Vieira and J. Messing, Waksman Institute of Microbiology, Rutgers, Piscataway, NJ). The 2.3 kb asFXIII insert was isolated from pUC18 #9 by digestion with Pst I, subcloned into the pUC118 Pst I site and transformed into E. coli strain JM109. A positive clone, which contained the 2.3 kb asFXIII insert in the anti-sense orientation, was designated pRS201.

The 120 bp 3′ untranslated region of the asFXIII cDNA was removed and an Xho I site inserted 3′ to the translation stop codon by site directed mutagenesis. Plasmid pRS201 was transformed into E. coli strain MV1193 and single-stranded template DNA was isolated. Oligonucleotide ZC1113 (Table 1) was designed to remove the 3′ untranslated region following the asFXIII coding sequence and to introduce an Xho I site 3′ to the translation stop. The single-stranded template of pRS201 was subjected to in vitro mutagenesis by the method of Zoller and Smith (1984, ibid.) using the mutagenic oligonucleotide ZC1113. A positive clone, confirmed by restriction analysis, was designated pRS202.


B. Construction of Plasmid pRS217


As shown in Figure 9, the 2.2 kb asFXIII cDNA fragment from plasmid pRS202 was placed behind the ADH2-4ᶜ promoter and placed in plasmid pEAS102 (Example IV). Plasmid pTRK4c (Example III.B.) was digested to completion with Eco RI and Sal I to isolate the 4 kb fragment comprising the ADH2-4ᶜ promoter, the TPI1 terminator and the pIC7RI* vector sequences. Oligonucleotides ZC1056 and ZC1057 (Table 1) were designed to form an adaptor with an Eco RI adhesive end, a Bgl II site and a Pst I adhesive end. The Eco RI adhesive end of the adapter, upon ligation to another Eco RI adhesive end, destroys the Eco RI site. Oligonucleotides ZC1056 and ZC1057 were kinased and annealed to form the Eco RI adaptor. The 2.2 kb Pst I-Xho I asFXIII fragment, isolated from plasmid pRS202 (Example V.A.) was joined with the ZC1056/1057 adaptor and the 4 kb pTRK4c fragment in a three-part ligation. The resultant plasmid was designated pRS215.

The expression unit present in pRS215 was placed in the yeast vector pEAS102. Plasmid pEAS102 was digested to completion with Hind III followed by treatment with bacterial alkaline phosphatase to prevent recircularization. Plasmid pRS215 was digested with Hind III to isolate the 3.5 kb expression unit comprising the ADH2-4ᶜ promoter, the asFXIII cDNA and the TPI1 terminator. These two fragments were ligated together to create plasmid pRS217.

Plasmid pRS217 was transformed into Saccharomyces cerevisiae strain XV794-7-1C (MATa ade2-1 leu2-3 leu2-113 ura3 Δpep4::TPI1-CAT cir±) using the method essentially described by Beggs (Nature 275:104-108, 1978). Transformants were selected for the ability to grow on synthetic medium lacking uracil (-UraD).

Expression of the Factor XIII a subunit from the pRS217 plasmid transformed into the strain XV794-7-1C was achieved by first growing the transformants overnight in 5 ml -UraD. The overnight cultures were innoculated into 1 liter -UraD and grown for 48 hours at 30°C. The cultures were centrifuged to harvest the cell pellets, which were washed once with distilled water and stored at -80°C.

Cell pellets were assayed for active Factor XIII a subunit as described in Example XI. XV794-7-1C cells transformed with pRS217 produced 50 mg/l Factor XIII a subunit.

C. Construction of Plasmid pRS216

As shown in Figure 9, the 2.2 kb asFXIII cDNA fragment from plasmid pRS202 was placed behind the TPI1 promoter and inserted into plasmid pEAS102 (Example IV). Plasmid pMVR1 (Example III.A.) was digested to completion with Eco RI and Sal I to isolate the 3.7 kb fragment comprising the TPI1 promoter, the TPI1 terminator and the pIC7RI* vector sequences. Plasmid pRS202 (Example V.A.) was digested with Pst I and Xho I to isolate the 2.2 kb asFXIII fragment. The 2.2 kb asFXIII fragment, the kinased and annealed ZC1056/ZC1057 adaptor (Example V.B.) and the linearized pMVR1 were joined in a three-part ligation to create plasmid pRS214.

The expression unit present in pRS214 was placed in the yeast vector pEAS102. Plasmid pEAS102 was linearized by digestion with Hind III and treated with bacterial alkaline phosphatase to prevent recircularization. Plasmid pRS214 was digested with Hind III to isolate the 3.2 kb expression unit comprising the TPI1 promoter, the asFXIII cDNA and the TPI1 terminator. These two fragments were ligated together to create the plasmid pRS216.

Yeast host cells were transformed and grown as described above. XV797-7-1C transformed with pRS216 produced 10 mg/l active Factor XIII a subunit as assayed in Example XI.


EXAMPLE VII

Secretion of the Factor XIII a Subunit from Yeast

A. Construction of Plasmid pRS203

To construct expression units which are capable of directing the secretion of FXIII a subunit protein, the asFXIII cDNA was altered to insert a Hind III site 3′ to the translation start site for the insertion of secretion signal sequences. Oliogonucleotide ZC1206 (Table 1) was designed to remove the 19 bp of 5′ untranslated DNA and insert a Hind III site 3′ to the translation start site. Single-stranded template DNA was made from E. coli strain MV1193 transformed with pRS202 (Example V.A.). The template was subjected to in vitro mutagenesis using ZC1206 and the method described by Zoller and Smith (1984, ibid.). A positive clone, which was confirmed by restriction analysis, was designated pRS203 (Figure 8).

B. Construction of Optimized MFα1

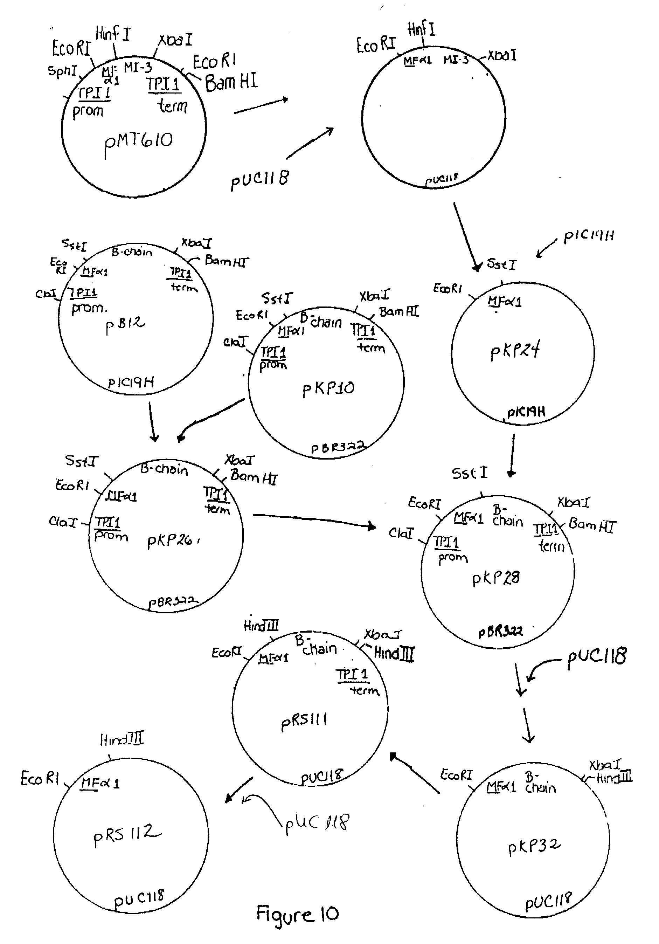

The codon-optimized MFα1 signal peptide sequence was obtained from an expression vector containing the gene for the insulin precursor B(1-29)-Ala-Ala-Lys-A(1-21) (Markussen et al., EP 164,529). An Eco RI-Xba I fragment comprising the MFa1 signal peptide and insulin sequences from pMT610 (Markussen et al., ibid.) was cloned into Eco RI, Xba I digested pUC118 (Figure 10) and single-stranded template DNA was prepared. This template was then mutagenized using the method described by Zoller and Smith (1984), ibid.), using the mutagenic olionucleotide ZC862 (Table 1). The mutagenesis resulted in the creation of an Sst I site at the 3′ end of the MFα1 signal peptide sequence. A positive clone containing an Sst I site 3′ to the MFα1 signal peptide sequence, was selected and designated pKP23. The MFα1 signal peptide sequence was removed from plasmid pKP23 and subcloned into pIC19H (Marsh et al., Gene 32:481-485, 1984). The resultant plasmid was designated pKP24.

Plasmid pB12, comprising the TPI1 promoter; the MFα1 signal peptide sequence; a DNA sequence encoding human PDGF B-chain; the TPI1 terminator and the pIC19H vector sequences, was digested with Eco RI and Xba I to isolate the fragment encoding the MFα1 signal peptide and PDGF B-chain. Plasmid pKP10, comprising the TPI1 promoter-MFα1 signal sequence-V-SIS-TPI1 terminator expression unit inserted into a pBR322 vector lacking an Eco RI site, was digested with Eco RI and Xba I to remove the MFα1 signal sequence and the B chain sequence. The vector fragment of pKP10 was ligated with the fragment derived from plasmid pB12. The resultant plasmid pKP26, was digested with Eco RI and Sst I to isolate the fragment comprising the TPI1 promoter, the V-SIS sequence, the TPI1 terminator and the pBR322 vector sequences. Plasmid pKP24 was digested with Eco RI and Sst I to isolate the fragment encoding the codon-optimized MFα1 signal peptide sequence. The fragment encoding the codon-optimized MFα1 signal peptide and the Eco RI-Set I vector fragment of pKP26 were joined to create plasmid pKP28.

The Sst I site introduced into the MFα1 signal peptide sequence to facilitate the construction of pKP28 was then removed to restore the wild-type coding sequence. Plasmid pKP28 was digested with Eco RI and Xba I to isolate the fragment encoding the codon-optimized MFα1 signal peptide sequence and the PDGF B-chain. This fragment was cloned into pUC118 which had been linearized by digestion with Eco RI and

16

Xba I. Single-stranded template was isolated and the template was mutagenized by the method described by Zoller and Smith (1984, ibid.) using oligonucleotide ZC1019 (Table 1). A correctly mutagenized plasmid was designated pKP32.

The codon-optimized MFα1 signal peptide sequence present in plasmid pKP32 was modified by in vitro mutagenesis to introduce the natural Glu-Ala-Glu-Ala sequence and Hind III site found in the MFα1 signal sequence. Single-stranded template DNA, made from pKP32, was mutagenized using ZC1059 (Table 1) by the method described by Zoller and Smith (1984, ibid.). DNA from positive colonies was cut with Hind III to screen for the introduction of a Hind III site at the 3′ end of the signal sequence. The clones were then sequenced to further confirm the mutagenesis. A positive clone with the correct sequence for the codon optimized MFα1 signal peptide, containing the natural Glu-Ala-Glu-Ala sequence and Hind III site, was designated pRS111. The mutagenized MFα1 signal peptide was subcloned into the vector pUC118. Plasmid pRS111 was digested with Hind III to eliminate the B chain sequence. The plasmid was recircularized and designated pRS112 (Figure 10).

C. Construction of Vectors for the Secretion of the Factor XIII a Subunit from Yeast using the MFα1 Signal Peptide

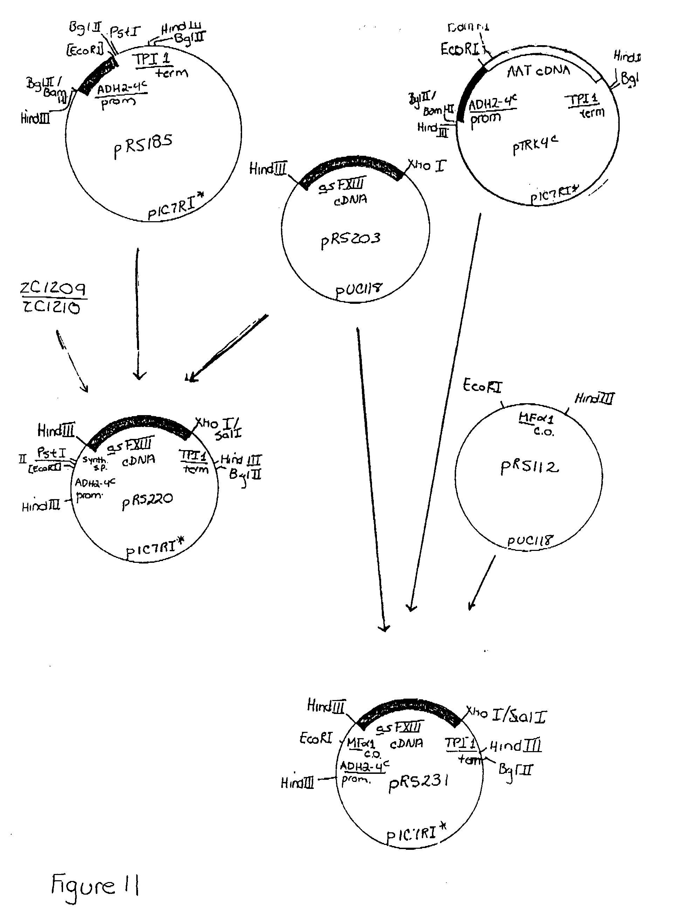

To construct an expression unit comprising the ADH2-4ᶜ promoter, the codon optimized MFα1 signal peptide, the asFXIII cDNA and the TPI1 terminator, plasmid pTRK4c was used as the source of the ADH2-4ᶜ promoter and TPI1 terminator. Plasmid pTRK4c was digested with Eco RI and Sal I to isolate the 4 kb fragment comprising the ADH2-4ᶜ promoter, the TPI1 terminator, and the pIC7RI* vector sequences. Plasmid pRS203 (Example VIII.A.) was digested with Hind III and Xho I to isolate the 2.2 kb asFXIII cDNA fragment. Plasmid pRS112 was digested with Eco RI and Hind III to isolate the 0.34 kb MFα1 promoter fragment. The pTRK4c fragment was joined with the asFXIII cDNA fragment and the MFα1 signal peptide in a three-part ligation. The resultant plasmid was designated pRS231 (Figure 11).

The expression unit present in plasmid pRS231 was inserted into the vectors YEp13 and pEAS102. Plasmid pRS231 was digested with Xho I to isolate the 5 kb expression unit. This fragment was ligated to YEp13 which had been linearized by digestion with Xho I. The resultant plasmid was designated pRS233. The 5 kb Xho I fragment isolated from pRS231 was ligated into pEAS102 which had been linearized by digestion with Sal I. The resultant plasmid was designated pRS232.

The above-described plasmids are transformed, essentially as described by Beggs (1978, ibid.), into a suitable yeast host, such as strain XV794-7-1C. Transformed cells are selected and maintained on synthetic medium lacking uracil(-UraD). Transformants are inoculated into 5 ml -UraD and are grown overnight at 30°C. The overnight culture is used to inoculate 1 l -UraD. The 1 l culture is grown as described above. The cultures are then centrifuged to remove the cells and the supernatant fractions are harvested. The supernatants are assayed for the presence of the Factor XIII a subunit using the method described in Example XI.B.

D. Construction of Vectors for the Secretion of Factor XIII a Subunit from Yeast Using a Synthetic Signal Peptide

An expression unit comprising the ADH2-4ᶜ promoter, a 19 amino acid signal peptide designed using the rules of von Heijne (ibid.), the asFXIII cDNA and the TPI1 terminator was constructed. Oligonucleotides ZC1209 and ZC1210 (Table 1) was designed to form, when annealed, an adaptor comprising a 5′ Pst I adhesive end, a sequence encoding a 19 amino acid signal peptide (with the amino acid sequence Met-Leu-Leu-Gln-Ala-Phe-Leu-Phe-Leu-Leu-Ala-Gly-Phe-Ala-Pro-Gly-Thr-Glu-Ala), and a 3′ Hind III adhesive end. Oligonucleotides were kinased and annealed using the method described by Maniatis et al. (Molecular Cloning: A Laboratory Manual, Cold Spring Harbor Laboratory, Cold Spring Harbor, N.Y., 1982).

The source of ADH2-4ᶜ promoter and the TPI1 terminator was plasmid pTRK4c (Example III.B.). Plasmid pTRK4c was modified to place a Pst I site 3′ to the ADH2-4ᶜ promoter. Plasmid pTRK4c was digested with Pst I and Eco RI to isolate the 3.7 kb fragment comprising the ADH2-4ᶜ promoter, the TPI1 terminator and the pIC7RI* vector sequences. The kinased, annealed ZC1056/ZC1057 (Example V.B.) adaptor was ligated with the pTRK4c fragment. The resultant plasmid was designated pRS185 (Figure 7). Plasmid pRS185 was linearized by digestion with Pst I and Sal I. Plasmid pRS203 (Example VII.A.) was digested with Hind III and Xho I to isolate the 2.2 kb asFXIII cDNA fragment which was joined with the linearized pRS185 and the kinased, annealed ZC1209/ZC1210 adaptor to create plasmid pRS220 (Figure 11).

17

The expression unit present in plasmid pRS220 is cloned into the vector pEAS102 (Example IV) to create plasmid pRS221. Plasmid pRS220 is digested with Xho I to isolate the 5 kb expression unit. Plasmid pEAS102 is digested with Sal I and treated with bacterial alkaline phophatase to prevent recircularization. The 5 kb expression unit is ligated with the linearized pEAS102 vector. The resultant plasmid, designated pE220, comprises the ADH2-4$\underline{c}$ promoter, the adapter encoding the 19 amino acid synthetic signal peptide, the asFXIII cDNA, the TPI1 terminator and the pEAS102 vector sequences.

Yeast cells are transformed and cultured as described above. The presence of Factor XIII a subunit is determined using the assay method described in Example XI.B.

## EXAMPLE IX

### Expression of the a′ Subunit of Factor XIII

The a subunit of Factor XIII is activated so the a′ subunit via a thrombin cleavage, which removes 37 amino acids from the amino-terminus of the protein. The asFXIII cDNA is altered to remove the sequences encoding the amino-terminal 37 amino acids. Single-stranded template DNA was prepared from plasmid pRS202 (Example V.A.) which comprises the asFXIII cDNA in the vector pUC118. An oligonucleotide is designed to remove the 5′ sequences encoding the 37 bp activation peptide and replace it with an Eco RI site followed by sequences encoding an intitiation methionine which is fused in-frame with GGC at +38 of the asFXIII cDNA coding region. This oligonucleotide is used to mutagenize the pRS202 single-stranded template DNA using the method described by Zoller and Smith (ibid.). The resultant sequence encodes the amino terminal portion of the a′ subunit (a′sFXIII) with an Eco RI site 5′ to the initiation methionine codon which is fused to the codon encoding amino acid +38. Replicative form DNA, prepared from the mutagenized phage, is digested from Eco RI and Xho I to isolate the a′sFXIII cDNA.

An expression vector comprising the ADH2-4$\underline{c}$ promoter, the a′sFXIII cDNA and the TPI1 terminator is constructed. The source of the ADH2-4$\underline{c}$ promoter and TPI1 terminator is plasmid pTRK4c (Example III.B.). Plasmid pTRK4c is digested with Eco RI and Sal I to isolate the 4 kb fragment comprising the ADH2-4$\underline{c}$ promoter, the TPI1 terminator and the pIC7RI* vector sequences. This linearized vector fragment is ligated with the Eco RI-Xho I a′sFXIII cDNA fragment. The resultant plasmid is designated pa′TRK4c.

The expression unit present in pa′TRK4c is placed in the yeast vector pEAS102. The a′sFXIII cDNA fragment is isolated from plasmid pa′TRK4c by digestion with Hind III. The a′sFXIII cDNA fragment is joined with plasmid pEAS102 linearized by digestion with Hind III and treated with bacterial alkaline phosphatase to prevent recircularization. The ligation mixture is transformed into E. coli strain RRI. Plasmid DNA, prepared from the resultant transformants, is analyzed by restriction analysis to determine the orientation of the insert. A positive clone is designated pa′EAS102.

Yeast cells are transformed and cultured as described above. Factor XIII a subunit is assayed using the method described in Example XI.B.

## EXAMPLE X

### Expression of the Factor XIII b Subunit

A. Expression in Yeast

Secretion of the b subunit of Factor XIII is achieved by linking the MFα1 signal peptide sequence to the coding sequence for the mature form of the b subunit of Factor XIII. The fusion between the codon-optimized MFα1 signal peptide sequence present in plasmid pKP24 (Example VIII.A.) and the Factor XIII b subunit cDNA (FXIIIb) involves in vitro mutagenesis. Plasmid pKP24, comprising the codon-optimized MFα1 signal peptide sequence with an Sst I site inserted after the codon encoding amino acid 81 of the MFα1 signal peptide sequence, is digested with Bam HI and Sst I to isolate the MFα1 signal peptide sequence. The 2.2 kb Eco RI FXIIIb cDNA fragment, isolated from the phage clone λHFXIIIb2.2, is subcloned into pUC118 which has been linearized by digestion with Eco RI. The ligation mixture is transformed into E. coli strain JM83. Plasmid DNA is made from the transformants and is digested with Stu I and Sst I to determine the orientation of the insert. A positive clone, designated pFXIIIb, comprises the FXIIIb cDNA in the anti-sense orientation with respect to the lacZ′ gene. Plasmid pFXIIIb is linearized by digestion with Bam HI and Sst I and ligated to the codon-optimized MFα1 signal peptide sequence fragment isolated from pKP24. The resultant plasmid is designated p118FXIIIb.

A synthetic oligonucleotide is designed to loop out the sequences between the Sst I site and the sequence encoding the mature FXIIIb subunit and insert the natural sequences encoding the amino acids 82-85 of MFα1. Plasmid p118FXIIIb is transformed into E. coli strain MV1193. Single-stranded template DNA, made from a transformant, is subjected to in vitro mutagenesis according to standard procedures. Positive clones are sequenced and analyzed by restriction analysis to confirm the mutagenesis. A correctly mutagenized clone is designated pMFFXIIIb.

The FXIIIb cDNA present in pMFFXIIIb is mutagenized using a synthetic oligonucleotide which will remove the 3′ flanking sequence and insert an Xba I site 3′ to the stop codon. Single-stranded template DNA is made from E. coli strain MV1193 transformed with pMFFXIIIb. The single-stranded template is subjected to in vitro mutagenesis. Positive clones are sequenced and analyzed by restriction analysis to confirm the mutagenesis. A correctly mutagenized clone is designated pMFFXIIIbX.

The insert present in plasmid pMFFXIIIbX is placed behind the TPI1 promoter present in plasmid pMVR1 (Example III.A.). Plasmid pMVR1 is digested with Eco RI and Xba I to isolate the 4 kb fragment comprising the TPI1 promoter, the TPI1 terminator and the pIC7RI* vector sequences. Plasmid pMFFXIIIbX is digested with Eco RI and Xba I to isolate the fragment comprising the MFα1 signal peptide sequence and the FXIIIb cDNA. This fragment is ligated with the pMVR1 fragment and the resultant plasmid is designated pMVR1FXIIIb.

The expression unit present in plasmid pMVR1FXIIIb is subcloned into the yeast vector YEp13. Plasmid pMVR1FXIIIb is digested with Sst I to isolate the 3.2 kb expression unit. This fragment was ligated to YEp13 which has been linearized by digestion with Sst I. The ligation mixture is transformed into E. coli strain RRI. Plasmid DNA, made from the transformants, is analyzed by restriction analysis to determine the orientation of the insert. Positive clones are designated pYEFXIIIb1, specifying a clone with the direction of transcription of the insert in the same orientation as the LEU2 gene, and pYEFXIIIb2, having the insert in the opposite orientation.

Plasmids pEFXIIIb1 and pYEFXIIIb2 are transformed into suitable yeast host cells and the cells are cultured as described above.

B. Expression of Factor XIIIb Subunit in Mammalian Cells

The b subunit of Factor XIII is expressed in mammalian cells by employing the Factor IX (FIX) leader sequence fused to the FXIIIb fragment (39 bp) in an expression vector derived from FIX/VII/pD2. The FIX leader sequence was obtained from FIX(-G) → pUC13, which was constructed as described below.

In order to obtain an appropriate secretory signal sequence, a cDNA coding for human Factor IX was obtained from a library made with mRNA from human liver (Kurachi and Davie, Proc. Natl. Acad. Sci. USA 79:6461-6464, 1982). The Factor IX sequence was isolated from the pBR322 vector by digestion with PstI and was inserted into the PstI site of pUC13. This plasmid was designated FIX-pUC13. In order to remove the G-rich region which was present at the 5′ end of the Factor IX insert as a result of cDNA cloning, a synthetic oligonucleotide adaptor was substituted for the 5′ end of the cloned fragment. Oligonucleotides ZC212 and ZC213 (Table 1) were synthesized and annealed to generate a 22 base pair overlap, the fragment ends filled in and cut with appropriate restriction endonucleases, and the resulting fragment was joined to the Factor IX sequence.

To construct the adaptor, 100 pmoles each of ZC212 and ZC213 were lyophilized and resuspended in 10 ul of 10x kinase/ligase buffer (600 mM Tris pH 8.0, 100 mM $MgCl_2$, 100 nM DTT) plus 86 ul $H_2O$. The annealing reaction was run at 65°C for 10 minutes; the mixture was slowly cooled to room temperature and put on ice. To this mixture was added 4 ul of 2.5 mM dNTP mix and 1 ul (8 units) $T_4$ DNA polymerase. The reaction was allowed to proceed 45 minutes at 14°C. Ten ul of 5 M $NH_4OAc$ was then added; and the DNA was extracted once with phenol/$CHCl_3$, twice with $CHCl_3$, and was precipitated with ethanol. The DNA was centrifuged and resuspended in 100 ul medium salt buffer (Maniatis et al., ibid., p. 100), digested with 9 units Pst I and 8 units Cfo I, and extracted as above.

The modified Factor IX sequence was then constructed by combining 0.16 pmole of the synthetic Pst I-Cfo I adaptor fragment, 0.14 pmole of a 1.4 kb Cfo I-Bam HI Factor IX fragment from FIX-pUC13, and 0.14 pmole of a 2.7 kb Bam HI-Pst I pUC13 vector fragment in a 20 ul reaction containing 60 mM Tris-HCl pH 7.5, 10 mM $MgCl_2$, 10 mM DTT, and 0.9 unit $T_4$ ligase. The reaction was incubated for 3 hours at room temperature and used to transform competent E. coli JM83 (Messing, Recombinant DNA Technical Bulletin, NIH publication No. 79-99, 2, No. 2, 43-48, 1979). The cells are plated with 50 ul of 2% X-gal (5 bromo-4-chloro-3 indolyl-β-D-galactoside) on L-broth containing 40 ug/ml ampicillin and incubated at 37°C overnight. White colonies were picked onto another plate containing ampicillin and grown at 37°C overnight. The colonies were blotted on Whatman 540 paper and the paper received for hybridization according to the method of Wallace et al. (Gene 16:21, 1981), except that the overnight incubation on chloramphenicol plates was omitted. The papers were incubated at 44 °C for 2 hours in 0.9 M NaCl, 0.09 M Tris-HCl pH 7.5, 6 mM EDTA, 0.5% Nonidet P-40, 150 ug/ml E. coli tRNA. The papers were probed with $^{32}$P-labeled ZC235 (Table 1), a 14-mer that is specific for the altered 5$'$ end sequence. Hybridization with 1-2 X 10$^6$ cpm per filter was carried out at 44 °C in the prehybridization buffer overnight. The filters were then washed three times in 6X SSC, 0.1% SDS at 4 °C and three times in 2X SCC, 0.1% SDS at 44 °C and exposed to X-ray film. Two positive clones are obtained. One of these clones was designated FIX(-G) → pUC13.

In order to confirm the sequence of the altered region of the Factor IX portion of the FIX(-G) → pUC13 construct, dideoxy sequencing directly on the pUC plasmid using the BRL reverse primer was performed using the method of Wallace et al. (Gene 16:21, 1981), using a primer end labeled with polynucleotide kinase and $\gamma^{32}$P ATP by the method of Chaconas et al. (Biochem. Biophys. Res. Comm. 60:962, 1975). The sequence was as predicted.

Plasmid FIX(-G) → pUC13 was digested with Pst I and Hae III to isolate the 39 bp FIX leader sequence. The FXIII b subunit is obtained from pFXIIIb. Plasmid pFXIIIb is linearized with Eco RI and the ends are blunted with the Klenow fragment of DNA polymerase. The DNA fragment is then digested with Hind III to isolate the 0.14 kb FXIIIb fragment. For ease of manipulation, the 39 bp FIX leader fragment and the 0.14 kb FXIIIb fragment are ligated into pUC12 linearized with Pst I and Hind III. The resultant plasmid is cleaved with Pst I and Hind III to isolate the 0.18 kb FIX-FXIIIb fusion fragment. This fragment is ligated into M13mp11 linearized with Pst I and Hind III.

The FIX leader sequence is precisely joined to FXIIIb by in vitro site-directed mutagenesis using an oligonucleotide designed to loop out the intervening sequences between the end of the FIX leader and the glutamic acid in the mature form of FXIIIb. The replicative form DNA is prepared from the mutagenized phage and is cut with Pst I and Hind III to isolate the 0.1 kb FIX-FXIIIb fusion fragment. For ease of manipulation, this fragment is subcloned into pUC13 linearized with Hind III and Pst I. The resultant plasmid is digested with Hind III and Bam HI to isolate the 0.1 kb FIX-FXIIIb fusion fragment.

λHFXIIIb2.2 is cut with Hind III and Eco RI to isolate the 2 kb 3$'$ end of FXIIIb. This fragment is subcloned into pUC13 linearized with Hind III and Eco RI. The resultant plasmid is linearized with Eco RI, the ends blunted with the Klenow fragment of DNA polymerase I and ligated to kinased Bgl II linkers. Excess Bgl II linkers are eliminated by digestion with Bgl II and the linear fragment is recircularized with T$_4$ DNA ligase. The resultant plasmid is cleaved with Hind III and Bgl II to isolate the 2 kb FXIIIb fragment.

The Factor XIIIb expression vector is derived from plasmid FIX/VII/pD2 (ATCC No. 53068). Digestion of FIX/VII/pD2 with Bam HI liberates the 5 kb FIX/VII fragment and leaves the pD2 vector fragment comprising the entire adenovirus 2 tripartite leader, the SV40 polyadenylation signal and pBR322 vector sequences. This vector fragment is treated with bacterial alkaline phosphatase to prevent recircularization and ligated in a three-part ligation to the 0.1 kb FIX-FXIIIB fusion fragment and the 2 kb FXIIIb fragment. The plasmid with the correct insert is determined by digestion with Bam HI and Hind III. This plasmid is designated pMFXIIIb.

The procedure used to transfect baby hamster kidney (BHK) cells (available from American Type Culture Collection, ATCC No. CCL10) with pMFXIIb is similar to published methods (for example, Wigler et al., Cell 14:725, 1978; Corsaro and Pearson, Somatic Cell Genetics 7:603, 1981; Graham and Van der Eb, Virol. 52:456, 1973). The BHK cells are grown at 37 °C, 5% CO$_2$, in Dulbecco's media (plus 10% heat-activating fetal calf serum and supplemented with glutamine and penicillin-streptomycin) in 60 mm tissue culture Petri dishes to a confluency of 20%. A total of 10 ug DNA is used to transfect one 60 mm dish: 3.75 ug of pMFXIIIb, 1.25 ug of pKO-neo (Southern and Berg, J. Mol. Appl. Genet. 1:327-341, 1982) and 5 ug of salmon sperm DNA. The DNAs are precipitated in 0.3 M NaOAc, 75% ethanol, rinsed with 70% ethanol and redissolved in 20 ul 10 mM Tris-HCl pH 8, 1 mM EDTA. The DNA is combined with 440 ul H$_2$O and 500 ul of 280 mM NaCl, 1.5 mM NaHPO$_4$, 12 mM dextrose, 50 mM HEPES pH 7.12. Sixty ul of 2 M CaCl$_2$ are added dropwise to the above mixture and the solution let stand at room temperature for 30 minutes. The solution is then added to the cells and the cells returned to 37 °C for 4 hours. The medium is removed and 5 ml of 20% DMSO in Dulbecco's with serum are added for 2 minutes at room temperature. The dish is then washed regularly with 2 changes of medium and incubated in fresh medium overnight. Twenty-four hours after the addition of the DNA, the medium is removed and selective medium added (10 mg/ml of G418, 498 ug/mg, Gibco, in Dulbecco's with serum). After 10 and 13 days, individual clones, representing cells that have incorporated the pKO-neo gene and are thus resistant to G418, are transferred to 96-well (or 24-well) plates and grown up for protein assays.

Cells are grown in Dulbecco's plus 10% fetal calf serum. The medium is separated from the cells and cellular debris by centrifugation, and assayed for Factor XIII subunit b polypeptide (e.g., by ELISA) and for biological activity. The cells are removed from the plates with trypsin, washed with fresh medium, centrifuged, and frozen at -20 °C. The cell pellets are then thawed in PBS, pelleted, and resuspended in PBS containing 0.25% Triton X-100. Samples are diluted and assayed for polypeptide and activity.

EXAMPLE XI

Description of Assays

A. Preparation of Cell Pellets

Appropriately grown yeast cells, as described in previous sections, were centrifuged to pellet the cells and the spent medium was discarded. The cells are pelleted and washed with distilled water. The washed pellets were frozen at -80°C before being assayed.

Crude glass bead lysates were made from the frozen cell pellets. The washed cell pellets were thawed on ice and diluted in an equal volume of phoshate buffered saline (PBS; Sigma, St. Louis, Mo.), 1 mM $\beta$-mercaptoethanol (Sigma). Glass beads (450-500 um) were added to one half the total volume. This mixture was vortexed at full speed for one minute, three times, with the samples cooled on ice between vortex bursts. The liquid was removed from the tubes with a pasteur pipet and transferred to a microfuge tube. The glass beads were washed one time in the original volume of PBS containing 1 mM $\beta$-mercaptoethanol (BME). The beads were vortexed one minute and the liquid was removed by pasteur pipet and pooled with the original lysate. The lysates were then centrifuged in an Eppendorf microfuge (Brinkmann, Westbury, N.Y.) at top speed for five minutes. The supernatant was carefully removed for assay.

B. Factor XIII Activity Assay

Factor XIII activity is measured using the method essentially described by Curtis and Lorand (ibid.). The lysates are measured for total yeast protein by the method described by Lowry et al. (J. Biol. Chem. 193:265-275, 1951). Samples are diluted to 10 ug/ul total yeast protein with PBS + 1 mM BME. Standards using commercially available Factor XIII (Table 2) are diluted in stepwise dilution in 50 mM Tris-HCl pH7.5, 10 mM dithiothreitol. Five ul of sample or standard are added to 35 ul 50 mM Tris-HCl pH7.6 and 1 ul thrombin (Table 2). The mixtures are allowed to incubate at 37°C for 30 minutes. The reactions are quenched by the addition of 2 U Hirudin (Sigma). Sixty ul Reaction Mixture (Table 2) 37°C are added and the mixture is incubated for 30 minutes. The reactions are stopped by adding 1 ml 7.5% trichloroacetic acid (TCA). The samples are spun in the microfuge at top speed for 5 minutes and the supernatants are discarded. The pellets are washed twice with 7.5% TCA. The pellets are then dissolved in 100 ul glacial acetic acid. Five ml scintillation cocktail (Optifluor, Packard Instruments Co., Downers Grove, Ill.) are added and the samples are counted on the scintillation counter (Beckman, Palo Alto, Calif.).

TABLE 2

| Thrombin: | Lyophilized thrombin (Sigma, St. Louis, Mo.) is dissolved in 50% Glycerol, .25 M Tris HCl pH 7.5 to 1000 U/ml. This solution is stored at -20°. |
|---|---|
| Commercial Factor XIII: | Factor XIII (Green Cross, Osaka, Japan) dissolved in 1 ml. 50% Glycerol, 50 mM Tris-HCl pH 7.5, 10 mM dithiothreitol (DTT; Sigma, St. Louis, Mo.). This solution is stored at -20°C. |
| N,N,Dimethylcasein: | N,N,Dimethylcasein (Sigma, St. Louis, Mo.) is dissolved to 10 mg/ml in 0.5 M Tris-HCl pH7.5 |
| $^3$H-Histamine Dihydrochloride: | 1 mCi $^3$H-Histamine dihydrochloride (New England Nuclear, Boston Mass.) was dissolved in 4 ml 10 mM unlabeled histamine dihydrochloride. This solution is stored at -20°C. |
| Reaction Buffer: | 1.4 M NaCl<br>1.0 M Tris Base<br>0.1 M CaCl$_2$<br>0.2 M DTT<br>pH adjusted to 7.5 |
| Reaction Mixture: | 2.0 ml $^3$H-Histamine dihydrochloride<br>8.0 ml N,N,Dimethylcasein<br>1.8 ml Reaction Buffer |

**Claims**

1. A DNA sequence that codes for a polypeptide that is functionally homologous to the a' subunit of Factor XIII, which sequence comprises sequence of Figure 1, from bp 202 to bp 2283.

2. A DNA sequence that codes for a polypeptide that is functionally homologous to the a' subunit of Factor XIII, which sequence comprises a DNA sequence coding for the amino acid sequence of Figure 1, starting with glycine, number 38, and ending with methionine, number 731.

3. An expression vector comprising a transcriptional promoter followed downstream by a DNA sequence according to Claim 1 or 2.

4. The expression vector of Claim 3, wherein said promoter is the ADH2-4c, TPI1, or GAL10.

5. A host cell transfected with an expression vector according to Claim 3 or 4.

6. The host cell of Claim 5 wherein said host cell is a yeast host cell.

7. A method of preparing a protein which, upon dissociation, has the biological activity of Factor XIIIa, comprising:
   introducing into a host cell an expression vector comprising a transcriptional promoter followed downstream by a DNA sequence that codes for a polypeptide that is functionally homologous to the a' subunit of Factor XIII comprising the amino acid sequence of Figure 1, starting with glycine, number 38, and ending with methionine, number 731 and a transcriptional promoter followed downstream by a DNA sequence that codes for a polypeptide that is functionally homologous to the b subunit of Factor XIII,
   culturing said host cell; and
   isolating the protein from said host cell.

8. A method of preparing a protein having the biological activity of Factor XIIIa, comprising:
   introducing into host cells an expression vector comprising a transcriptional promoter followed downstream by a DNA sequence that codes for a polypeptide that is functionally homologous to the a' subunit of Factor XIII comprising the amino acid sequence of Figure 1, starting with glycine, number 38, and ending with methionine, number 731;

culting said host cells;

isolating polypeptide from said host cells; and

dimerizing the polypeptide to form homodimers.

9. A method of preparing a protein having the biological activity of Factor XIIIa, comprising:

introducing into a host cell an expression vector comprising a transcriptional promoter followed downstream by a DNA sequence that codes for a polypeptide that is functionally homologous to the a' subunit of Factor XIII comprising the amino acid sequence of Figure 1, starting with glycine, number 38, and ending with methionine, number 731;

culturing said host cell; and

isolating homodimer from said host cell.

10. An expression vector comprising a transcriptional promoter followed downstream by a DNA sequence that codes for a protein having the structure and biological activity of human Factor XIII a subunit or the human Factor XIII a' subunit comprising the amino acid sequence of Figure 1, starting with glycine, number 38, and ending with methionine, number 731, wherein said DNA sequence is essentially free of 3' untranslated sequences normally associated with said DNA sequence.

11. A host cell transfected with an expression vector comprising a transcriptional promoter followed downstream by a DNA sequence that codes for a protein having the structure and biological activity of human Factor XIII a subunit or the human Factor XIII a' subunit comprising the amino acid sequence of Figure 1, starting with glycine, number 38, and ending with methionine, number 731, wherein said DNA sequence is essentially free of 3' untranslated sequences normally associated with said DNA sequence.

12. A method of preparing a protein having the biological activity of human Factor XIII, comprising: introducing into a host cell an expression vector comprising a transcriptional promoter followed downstream by a DNA sequence that codes for a protein having the structure and biological activity of human Factor XIII a subunit or the human Factor XIII a' subunit comprising the amino acid sequence of Figure 1, starting with glycine, number 38, and ending with methionine, number 731, wherein said DNA sequence is essentially free of 3' untranslated sequences normally associated with said DNA sequence; culturing said host cell; and isolating the protein encoded by said DNA sequence from said host cell.

**Patentansprüche**

1. Eine DNA-Sequenz, die für ein Polypeptid kodiert, das funktionell homolog zur a'-Untereinheit von Faktor XIII ist, wobei die Sequenz die Sequenz von Figur 1 von bp 202 bis bp 2283 umfaßt.

2. Eine DNA-Sequenz, die für ein Polypeptid kodiert, das funktionell homolog ist zu der a'-Untereinheit von Faktor XIII, wobei die Sequenz eine DNA-Sequenz umfaßt, die für die Aminosäuresequenz in Figur 1 kodiert, beginnend mit Glycin, Nummer 38, und endend mit Methionin, Nummer 731.

3. Ein Expressionsvektor, der einen Transkriptionspromotor umfaßt, dem flußabwärts eine DNA-Sequenz gemäß Anspruch 1 oder 2 folgt.

4. Der Expressionsvektor nach Anspruch 3, wobei besagter Promotor der ADH2-4c-, TPI1-, oder GAL10-Promotor ist.

5. Eine Wirtszelle, die mit einem Expressionsvektor nach Anspruch 3 oder 4 transfiziert ist.

6. Die Wirtszelle nach Anspruch 5, wobei besagte Wirtszelle eine Hefe-Wirtszelle ist.

7. Ein Verfahren zur Herstellung eines Proteins, das, bei Dissoziation, die biologische Aktivität von Faktor XIIIa besitzt, welches umfaßt:

Einführen eines Expressionsvektors, der einen Transkriptionspromotor, dem flußabwärts eine DNA-Sequenz folgt, die für ein Polypeptid kodiert, das funktionell homolog zur a'-Untereinheit von Faktor XIII ist, umfassend die Aminosäuresequenz von Figur 1, beginnend mit Glycin, Nummer 38, und endend mit Methionin, Nummer 731, und einen Transkriptionspromotor umfaßt, dem flußabwärts eine DNA-Sequenz folgt, die für ein Polypeptid kodiert, das funktionell homolog zur b-Untereinheit von Faktor XIII ist, in eine Wirtszelle,

Kultivieren besagter Wirtszelle; und

Isolieren des Proteins aus besagter Wirtszelle.

**8.** Ein Verfahren zur Herstellung eines Proteins mit der biologischen Aktivität von Faktor XIIIa, weiches umfaßt:

Einführen eines Expressionsvektors, der einen Transkriptionspromotor umfaßt, dem flußabwärts eine DNA-Sequenz folgt, die für ein Polypeptid kodiert, das funktionell homolog zur a'-Untereinheit von Faktor XIII ist, umfassend die Aminosäuresequenz von Figur 1, beginnend mit Glycin, Nummer 38, und endend mit Methionin, Nummer 731, in Wirtszellen;

Kultivieren besagter Wirtszellen;

Isolieren von Polypeptid aus besagten Wirtszeilen; und

Dimerisieren des Polypeptids, um Homodimere zu bilden.

**9.** Ein Verfahren zur Herstellung eines Proteins mit der biologischen Aktivität von Faktor XIIIa, welches umfaßt:

Einführen eines Expressionsvektors, der einen Transkriptionspromotor umfaßt, dem flußabwärts eine DNA-Sequenz folgt, die für ein Polypeptid kodiert, das funktionell homolog zur a'-Untereinheit von Faktor XIII ist, umfassend die Aminosäuresequenz von Figur 1, beginnend mit Glycin, Nummer 38, und endend mit Methionin, Nummer 731, in eine Wirtszelle;

Kultivieren besagter Wirtszelle; und

Isolieren von Homodimer aus besagter Wirtszelle.

**10.** Ein Expressionsvektor, der einen Transkriptionspromotor umfaßt, dem flußabwärts eine DNA-Sequenz folgt, die für ein Protein mit der Struktur und biologischen Aktivität der a-Untereinheit von menschlichem Faktor XIII oder der a'-Untereinheit von menschlichem Faktor XIII kodiert, umfassend die Aminosäuresequenz von Figur 1, beginnend mit Glycin, Nummer 38, und endend mit Methionin, Nummer 731, wobei besagte DNA-Sequenz im wesentlichen frei von 3' nicht-translatierten Sequenzen ist, die normalerweise mit besagter DNA-Sequenz assoziiert sind.

**11.** Eine Wirtszelle, die mit einem Expressionsvektor transfiziert ist, die einen Transkriptionspromotor umfaßt, dem flußabwärts eine DNA-Sequenz folgt, die für ein Protein mit der Struktur und biologischen Aktivität der a-Untereinheit von menschlichem Faktor XIII oder der a'-Untereinheit von menschlichem Faktor XIII kodiert, umfassend die Aminosäuresequenz von Figur 1, beginnend mit Glycin, Nummer 38, und endend mit Methionin, Nummer 731, wobei besagte DNA-Sequenz im wesentlichen frei von 3' nicht-translatierten Sequenzen ist, die normalerweise mit besagter DNA-Sequenz assoziiert sind.

**12.** Ein Verfahren zur Herstellung eines Proteins mit der biologischen Aktivität von menschlichem Faktor XIII, welches umfaßt: Einführen eines Expressionsvektors, der einen Transkriptionspromotor umfaßt, dem flußabwärts eine DNA-Sequenz folgt, die für ein Protein mit der Struktur und biologischen Aktivität der a-Untereinheit von menschlichem Faktor XIII oder der a'- Untereinheit von menschlichem Faktor XIII kodiert, umfassend die Aminosäuresequenz von Figur 1, beginnend mit Glycin, Nummer 38, und endend mit Methionin, Nummer 731, in eine Wirtszelle, wobei besagte DNA-Sequenz im wesentlichen frei von 3' nicht-translatierten Sequenzen ist, die normalerweise mit besagter DNA-Sequenz assoziiert sind; Kultivieren besagter Wirtszelle; und Isolieren des von besagter DNA-Sequenz kodierten Proteins aus besagter Wirtszelle.

## Revendications

**1.** Séquence d'ADN qui code pour un polypeptide qui est fonctionnellement homologue à la sous-unité a' du Facteur XIII, séquence qui comprend la séquence de la figure 1, allant de pb 202 à pb 2283.

**2.** Séquence d'ADN qui code pour un polypeptide qui est fonctionnellement homologue à la sous-unité a' du Facteur XIII, séquence qui comprend une séquence d'ADN codant pour la séquence d'aminoacides de la figure 1, commençant par la glycine, numéro 38, et se terminant par la méthionine, numéro 731.

**3.** Vecteur d'expression comprenant un promoteur de transcription suivi en aval par une séquence d'ADN suivant la revendication 1 ou 2.

24

4. Vecteur d'expression suivant la revendication 3, dans lequel le promoteur est le promoteur ADH2-46 TPI1, ou GAL10.

5. Cellule hôte transfectée avec un vecteur d'expression suivant la revendication 3 ou 4.

6. Cellule hôte suivant la revendication 5, qui est une cellule hôte de levure.

7. Procédé de préparation d'une protéine qui, par dissociation, possède l'activité biologique du Facteur XIIIa, comprenant :
   l'introduction dans une cellule hôte d'un vecteur d'expression comprenant un promoteur de transcription suivi en aval par une séquence d'ADN qui code pour un polypeptide qui est fonctionnellement homologue à la sous-unité a' du Facteur XIII comprenant la séquence d'aminoacides de la figure 1, commençant par la glycine, numéro 38, et se terminant par la méthionine, numéro 731, et un promoteur de transcription suivi en aval par une séquence d'ADN qui code pour un polypeptide qui est fonctionnellement homologue à une sous-unité b du Facteur XIII ;
   la culture de ladite cellule hôte ; et
   l'isolement de la protéine de ladite cellule hôte.

8. Procédé de préparation d'une protéine ayant l'activité biologique du Facteur XIIIa, comprenant :
   l'introduction dans des cellules hôtes d'un vecteur d'expression comprenant un promoteur de transcription suivi en aval par une séquence d'ADN qui code pour un polypeptide qui est fonctionnellement homologue à la sous-unité a' du Facteur XIII comprenant la séquence d'aminoacides de la figure 1, commençant par la glycine, numéro 38, et se terminant par la méthionine, numéro 731 ;
   la culture desdites cellules hôtes ;
   l'isolement du polypeptide desdites cellules hôtes ; et
   la dimérisation du polypeptide pour former des homodimères.

9. Procédé de préparation d'une protéine ayant l'activité biologique du Facteur XIIIa, comprenant :
   l'introduction dans une cellule hôte d'un vecteur d'expression comprenant un promoteur de transcription suivi en aval par une séquence d'ADN qui code pour un polypeptide qui est fonctionnellement homologue à la sous-unité a' du Facteur XIII comprenant la séquence d'aminoacides de la figure 1, commençant par la glycine, numéro 38, et se terminant par la méthionine, numéro 731 ;
   la culture de ladite cellule hôte ; et
   l'isolement de l'homodimère de ladite cellule hôte.

10. Vecteur d'expression comprenant un promoteur de transcription suivi en aval par une séquence d'ADN qui code pour une protéine ayant la structure et l'activité biologique de la sous-unité a du Facteur XIII humain ou de la sous-unité a' du Facteur XIII humain comprenant la séquence d'aminoacides de la figure 1, commençant par la glycine, numéro 38, et se terminant par la méthionine, numéro 731, ladite séquence d'ADN étant pratiquement dépourvue de séquences non traduites en 3' associées normalement à ladite séquence d'ADN.

11. Cellule hôte transfectée avec un vecteur d'expression comprenant un promoteur de transcription suivi en aval par une séquence d'ADN qui code pour une protéine ayant la structure et l'activité biologique de la sous-unité a du Facteur XIII humain ou de la sous-unité a' du Facteur XIII humain comprenant la séquence d'aminoacides de la figure 1, commençant par la glycine, numéro 38, et se terminant par la méthionine, numéro 731, ladite séquence d'ADN étant pratiquement dépourvue de séquences non traduites en 3' normalement associées à ladite séquence d'ADN.

12. Procédé de préparation d'une protéine ayant l'activité biologique du Facteur XIII humain, comprenant :
   l'introduction dans une cellule hôte d'un vecteur d'expression comprenant un promoteur de transcription suivi en aval par une séquence d'ADN qui code pour une protéine ayant la structure et l'activité biologique de la sous-unité a du Facteur XIII humain ou de la sous-unité a' du Facteur XIII comprenant la séquence d'aminoacides de la figure 1, commençant par la glycine, numéro 38, et se terminant par la méthionine, numéro 731, ladite séquence d'ADN étant pratiquement dépourvue de séquences non traduites en 3' normalement associées à ladite séquence d'ADN ; la culture de ladite cellule hôte ; et l'isolement de la protéine codée par ladite séquence d'ADN de ladite cellule hôte.

Fig. 1

Fig. 2

```
F.XIII b    R4  189   -----TKLX--CSSLRLI-E-NC-----YF---HPVKQTY---
            R5  248   ----EGR-RMRCP-PPP-LPINSKIQTHS---------ITYR--
            R6  308   ---IEGQEKVACE-EPPFIE-NGAANLHS--------KIYYN--
P2-GP I     R1    1   -----GRI---CP-XPDOLP-FS-TVVPL--------KTFYEP-
            R2   61   ----TPR--V-CP-FAGILE-NGA--VRY------TTFEYP-
            R3  119   -------APIICP-PPSIPT-F-ATLRVYXPSAGNNSL-YR--
            R4  182   ------RE-VKCP-FPSRPO-NGF--VNYPAXP---TLYYK--
            R5  242   ------KAS--CX-VPVKKA----TVV-YQGERVKIQEKFKNG
Factor B    R1    1   TPWSLARPQGSCS-LEG-VEIKGGS-----------FRLL
            R2   74   ------RA-IHCP-RPHDFE-NGE----YNPR---SPYYNV-
            R3  134   ---DNG-R-GYCS-NPGI-PI-GTRKMG--------SQ-YRL-
Haptoglobin       4   GNDVTDIADOGCP-KPPEIA-HG----------------YVE-
```

```
-EEGDVVQFFCHENYYLS--GSDLI-QCYNFG-WY---------PESPVC
--HGEIVHIECELNFEIH--GSAEIR-CE-DGKWT---------EP--PKC
---GDXVTYACKSGYLLH--GSNEI-TCN-RGKWT---------LP--PEC
---DEEITYSCKPGYVSR--GGMRKFICPLTGLWP--------INTLKC
----NTISFSCNTGFYLN--G-ADCAKCTEEGKWS--------PELPVC
----DTAVFECLPQHAMF--GNOTI-IRTTHGNWT--------KLPEC
----DXAIFGCHOGYSLO--GPEEI-ECTKLGNWS--------AMPSC
MLHGDXVSFFCXNKEKKC--SYTEDAQCI-DG--T--------IEVPKC
QE-GDALEYVCPSCFYPY--PVQT-RTCRSTGSWSTLKTQDQKTVRKAEC
---SDEISFHCYDGYTLR--GSAN-RTCQVNGRWS--------GQTAIC
---EDSVIYHCSRGLTLR--GSQR-RTCQEGGSWS--------GTEPSC
----HSVRYQCKNYYKLRTEG-DGVYULNNEKQWI----NKAVGDKLPEC
```

FIG.3

Figure 4

EP 0 268 772 B1

Figure 5

Figure 6

31

Figure 7

Figure 8

Figure 9

Figure 10

Figure 11